# EUROPEAN PATENT APPLICATION

(11) **EP 2 267 153 A1**
(43) Date of publication of application: **29.12.2010**
(21) Application number: 09161122.8
(22) Date of filing: 26.05.2009
(51) Int. Cl.: C12Q 1/68, G01N 33/574, C07K 14/47, C12N 15/86, A01K 67/027

(54) **Identification of netrin-1 receptor unc5c gene mutation in solid cancers**

(71) Applicant: Universite Claude Bernard Lyon 1, 69100 Villeurbanne (FR); Centre National pour la Recherche Scientifique (CNRS), 75016 Paris (FR)
(72) Inventor: Mehlen, Patrick, 69360 Serezin du Rhône (FR); Coissieux, Marie-May, 69003 Lyon (FR); Bernet, Agnès, 69740 Genas (FR)
(74) Representative: Warcoin, Jacques

(57) **Abstract**

The present invention relates to the identification of the UNC5C A628K, P57T, R603C, Q630C, A841Y or A628E variant implicated in solid cancer. A method is provided for the detection of these variants as shown in SEQ ID No 1 or 5 in solid cancers and to a screening test for predisposition to solid cancers, such as colorectal cancers.

SEQUENCE LISTING

<110>  Centre National de la Recherche Scientifique (CNRS)
       Universite Claude Bernard Lyon 1

<120>  Identification of netrin-1 receptor UNC5C gene mutation in solid
       cancers

<130>  67889

<160>  14

<170>  PatentIn version 3.3

<210>  1
<211>  931
<212>  PRT
<213>  homo sapiens


<220>
<221>  misc_feature
<223>  Mutated UNC5C A628K

<400>  1

Met Arg Lys Gly Leu Arg Ala Thr Ala Ala Arg Cys Gly Leu Gly Leu
1               5                   10                  15


Gly Tyr Leu Leu Gln Met Leu Val Leu Pro Ala Leu Ala Leu Leu Ser
            20                  25                  30


Ala Ser Gly Thr Gly Ser Ala Ala Gln Asp Asp Asp Phe Phe His Glu
        35                  40                  45


Leu Pro Glu Thr Phe Pro Ser Asp Pro Pro Glu Pro Leu Pro His Phe
    50                  55                  60


Leu Ile Glu Pro Glu Glu Ala Tyr Ile Val Lys Asn Lys Pro Val Asn
65                  70                  75                  80


Leu Tyr Cys Lys Ala Ser Pro Ala Thr Gln Ile Tyr Phe Lys Cys Asn
                85                  90                  95


Ser Glu Trp Val His Gln Lys Asp His Ile Val Asp Glu Arg Val Asp
            100                 105                 110


Glu Thr Ser Gly Leu Ile Val Arg Glu Val Ser Ile Glu Ile Ser Arg
        115                 120                 125


Gln Gln Val Glu Glu Leu Phe Gly Pro Glu Asp Tyr Trp Cys Gln Cys
    130                 135                 140

Val Ala Trp Ser Ser Ala Gly Thr Thr Lys Ser Arg Lys Ala Tyr Val
145                 150                 155                 160

Arg Ile Ala Tyr Leu Arg Lys Thr Phe Glu Gln Glu Pro Leu Gly Lys
                165                 170                 175

Glu Val Ser Leu Glu Gln Glu Val Leu Leu Gln Cys Arg Pro Pro Glu
            180                 185                 190

Gly Ile Pro Val Ala Glu Val Glu Trp Leu Lys Asn Glu Asp Ile Ile
        195                 200                 205

Asp Pro Val Glu Asp Arg Asn Phe Tyr Ile Thr Ile Asp His Asn Leu
    210                 215                 220

Ile Ile Lys Gln Ala Arg Leu Ser Asp Thr Ala Asn Tyr Thr Cys Val
225                 230                 235                 240

Ala Lys Asn Ile Val Ala Lys Arg Lys Ser Thr Thr Ala Thr Val Ile
                245                 250                 255

Val Tyr Val Asn Gly Gly Trp Ser Thr Trp Thr Glu Trp Ser Val Cys
            260                 265                 270

Asn Ser Arg Cys Gly Arg Gly Tyr Gln Lys Arg Thr Arg Thr Cys Thr
        275                 280                 285

Asn Pro Ala Pro Leu Asn Gly Gly Ala Phe Cys Glu Gly Gln Ser Val
    290                 295                 300

Gln Lys Ile Ala Cys Thr Thr Leu Cys Pro Val Asp Gly Arg Trp Thr
305                 310                 315                 320

Pro Trp Ser Lys Trp Ser Thr Cys Gly Thr Glu Cys Thr His Trp Arg
                325                 330                 335

Arg Arg Glu Cys Thr Ala Pro Ala Pro Lys Asn Gly Gly Lys Asp Cys
            340                 345                 350

Asp Gly Leu Val Leu Gln Ser Lys Asn Cys Thr Asp Gly Leu Cys Met
        355                 360                 365

Gln Thr Ala Pro Asp Ser Asp Asp Val Ala Leu Tyr Val Gly Ile Val
    370                 375                 380

Ile Ala Val Ile Val Cys Leu Ala Ile Ser Val Val Val Ala Leu Phe
385                 390                 395                 400

Val Tyr Arg Lys Asn His Arg Asp Phe Glu Ser Asp Ile Ile Asp Ser
                405                 410                 415

Ser Ala Leu Asn Gly Gly Phe Gln Pro Val Asn Ile Lys Ala Ala Arg
            420                 425                 430

Gln Asp Leu Leu Ala Val Pro Pro Asp Leu Thr Ser Ala Ala Ala Met
        435                 440                 445

Tyr Arg Gly Pro Val Tyr Ala Leu His Asp Val Ser Asp Lys Ile Pro
    450                 455                 460

Met Thr Asn Ser Pro Ile Leu Asp Pro Leu Pro Asn Leu Lys Ile Lys
465                 470                 475                 480

Val Tyr Asn Thr Ser Gly Ala Val Thr Pro Gln Asp Asp Leu Ser Glu
                485                 490                 495

Phe Thr Ser Lys Leu Ser Pro Gln Met Thr Gln Ser Leu Leu Glu Asn
            500                 505                 510

Glu Ala Leu Ser Leu Lys Asn Gln Ser Leu Ala Arg Gln Thr Asp Pro
        515                 520                 525

Ser Cys Thr Ala Phe Gly Ser Phe Asn Ser Leu Gly Gly His Leu Ile
    530                 535                 540

Val Pro Asn Ser Gly Val Ser Leu Leu Ile Pro Ala Gly Ala Ile Pro
545                 550                 555                 560

Gln Gly Arg Val Tyr Glu Met Tyr Val Thr Val His Arg Lys Glu Thr
                565                 570                 575

Met Arg Pro Pro Met Asp Asp Ser Gln Thr Leu Leu Thr Pro Val Val
            580                 585                 590

Ser Cys Gly Pro Pro Gly Ala Leu Leu Thr Arg Pro Val Val Leu Thr
        595                 600                 605

Met His His Cys Ala Asp Pro Asn Thr Glu Asp Trp Lys Ile Leu Leu
    610                 615                 620

Lys Asn Gln Lys Ala Gln Gly Gln Trp Glu Asp Val Val Val Val Gly

625                 630                 635                 640

Glu Glu Asn Phe Thr Thr Pro Cys Tyr Ile Lys Leu Asp Ala Glu Ala
                645                 650                 655

Cys His Ile Leu Thr Glu Asn Leu Ser Thr Tyr Ala Leu Val Gly His
            660                 665                 670

Ser Thr Thr Lys Ala Ala Ala Lys Arg Leu Lys Leu Ala Ile Phe Gly
        675                 680                 685

Pro Leu Cys Cys Ser Ser Leu Glu Tyr Ser Ile Arg Val Tyr Cys Leu
    690                 695                 700

Asp Asp Thr Gln Asp Ala Leu Lys Glu Ile Leu His Leu Glu Arg Gln
705                 710                 715                 720

Thr Gly Gly Gln Leu Leu Glu Glu Pro Lys Ala Leu His Phe Lys Gly
                725                 730                 735

Ser Thr His Asn Leu Arg Leu Ser Ile His Asp Ile Ala His Ser Leu
            740                 745                 750

Trp Lys Ser Lys Leu Leu Ala Lys Tyr Gln Glu Ile Pro Phe Tyr His
        755                 760                 765

Val Trp Ser Gly Ser Gln Arg Asn Leu His Cys Thr Phe Thr Leu Glu
    770                 775                 780

Arg Phe Ser Leu Asn Thr Val Glu Leu Val Cys Lys Leu Cys Val Arg
785                 790                 795                 800

Gln Val Glu Gly Glu Gly Gln Ile Phe Gln Leu Asn Cys Thr Val Ser
                805                 810                 815

Glu Glu Pro Thr Gly Ile Asp Leu Pro Leu Leu Asp Pro Ala Asn Thr
            820                 825                 830

Ile Thr Thr Val Thr Gly Pro Ser Ala Phe Ser Ile Pro Leu Pro Ile
        835                 840                 845

Arg Gln Lys Leu Cys Ser Ser Leu Asp Ala Pro Gln Thr Arg Gly His
    850                 855                 860

Asp Trp Arg Met Leu Ala His Lys Leu Asn Leu Asp Arg Tyr Leu Asn
865                 870                 875                 880

Tyr Phe Ala Thr Lys Ser Ser Pro Thr Gly Val Ile Leu Asp Leu Trp
                885                 890                 895

Glu Ala Gln Asn Phe Pro Asp Gly Asn Leu Ser Met Leu Ala Ala Val
            900                 905                 910

Leu Glu Glu Met Gly Arg His Glu Thr Val Val Ser Leu Ala Ala Glu
        915                 920                 925

Gly Gln Tyr
    930

<210>  2
<211>  2796
<212>  DNA
<213>  homo sapiens

<220>
<221>  misc_feature
<223>  Mutated Human UNC5C5 coding sequence comprising the
       1882_1883GC>AA mutation

<400>  2
atgaggaaag gtctgcgggc gacagcggcc cgctgcggac tgggactggg atacttgctg      60

caaatgctcg tgctacctgc cctggccctg ctcagcgcca gcggcactgg ctccgccgcc     120

caagatgatg acttttttca tgaactccca gaaacttttc cttctgatcc acctgagcct     180

ctgccacatt tccttattga gcctgaagaa gcttatattg tgaagaataa gcctgtgaac     240

ctgtactgta aagcaagccc tgccacccag atctatttca agtgtaatag tgaatgggtt     300

catcagaagg accacatagt agatgaaaga gtagatgaaa cttccggtct cattgtccgg     360

gaagtgagca ttgagatttc gcgccagcaa gtggaagaac tctttggacc tgaagattac     420

tggtgccagt gtgtggcctg gagctccgcg ggtaccacaa agagccggaa ggcgtatgtg     480

cgcattgcat atctacggaa gacatttgag caggaacccc taggaaagga agtgtctttg     540

gaacaggaag tcttactcca gtgtcgacca cctgaaggga tcccagtggc tgaggtggaa     600

tggttgaaaa atgaagacat aattgatccc gttgaagatc ggaattttta tattactatt     660

gatcacaacc tcatcataaa gcaggcccga ctctctgata ctgcaaatta cacctgtgtt     720

gccaaaaaca ttgttgccaa gaggaaaagt acaactgcca ctgtcatagt ctatgtcaac     780

ggtggctggt ccacctggac ggagtggtct gtgtgtaaca gccgctgtgg acgagggtat     840

cagaaacgta caaggacttg taccaacccg gcaccactca atgggggtgc cttctgtgaa     900

gggcagagtg tgcagaaaat agcctgtact acgttatgcc cagtggatgg caggtggacg     960

ccatggagca agtggtctac ttgtggaact gagtgcaccc actggcgcag gagggagtgc 1020

acggcgccag cccccaagaa tggaggcaag gactgcgacg gcctcgtctt gcaatccaag 1080

aactgcactg atgggctttg catgcagact gctcctgatt cagatgatgt tgctctctat 1140

gttgggattg tgatagcagt gatcgtttgc ctggcgatct ctgtagttgt ggccttgttt 1200

gtgtatcgga agaatcatcg tgactttgag tcagatatta ttgactcttc ggcactcaat 1260

gggggctttc agcctgtgaa catcaaggca gcaagacaag atctgctggc tgtaccccca 1320

gacctcacgt cagctgcagc catgtacaga ggacctgtct atgccctgca tgacgtctca 1380

gacaaaatcc caatgaccaa ctctccaatt ctggatccac tgcccaacct gaaaatcaaa 1440

gtgtacaaca cctcaggtgc tgtcaccccc caagatgacc tctctgagtt tacgtccaag 1500

ctgtcccctc agatgaccca gtcgttgttg gagaatgaag ccctcagcct gaagaaccag 1560

agtctagcaa ggcagactga tccatcctgt accgcatttg gcagcttcaa ctcgctggga 1620

ggtcacctta ttgttcccaa ttcaggagtc agcttgctga ttcccgctgg ggccattccc 1680

caagggagag tctacgaaat gtatgtgact gtacacagga aagaaactat gaggccaccc 1740

atggatgact ctcagacact tttgacccct gtggtgagct gtgggccccc aggagctctg 1800

ctcacccgcc ccgtcgtcct cactatgcat cactgcgcag accccaatac cgaggactgg 1860

aaaatactgc tcaagaacca gaaagcacag ggacagtggg aggatgtggt ggtggtcggg 1920

gaggaaaact tcaccacccc ctgctacatt aagctggatg cagaggcctg ccacatcctc 1980

acagagaacc tcagcaccta cgccctggta ggacattcca ccaccaaagc ggctgcaaag 2040

cgcctcaagc tggccatctt tgggcccctg tgctgctcct cgctggagta cagcatccga 2100

gtctactgtc tggatgacac ccaggatgcc ctgaaggaaa ttttacatct tgagagacag 2160

acgggaggac agctcctaga agaacctaag gctcttcatt ttaaaggcag cacccacaac 2220

ctgcgcctgt caattcacga tatcgcccat tccctctgga agagcaaatt gctggctaaa 2280

tatcaggaaa ttccatttta ccatgtttgg agtggatctc aaagaaacct gcactgcacc 2340

ttcactctgg aaagatttag cctgaacaca gtggagctgg tttgcaaact ctgtgtgcgg 2400

caggtggaag gagaagggca gatcttccag ctcaactgca ccgtgtcaga ggaacctact 2460

ggcatcgatt tgccgctgct ggatcctgcg aacaccatca ccacggtcac ggggcccagt 2520

gctttcagca tccctctccc tatccggcag aagctctgta gcagcctgga tgccccccag 2580

acgagaggcc atgactggag gatgctggcc cataagctga acctggacag gtacttgaat 2640

tactttgcca ccaaatccag cccaactggc gtaatcctgg atctttggga agcacagaac 2700

ttcccagatg gaaacctgag catgctggca gctgtcttgg aagaaatggg aagacatgaa 2760

acggtggtgt ccttagcagc agaagggcag tattaa 2796

<210> 3
<211> 3646
<212> DNA
<213> homo sapiens

<220>
<221> misc_feature
<223> Wild type UNC5C human gene sequence - GENEBANK NCBI NM_003728.2

<400> 3
ctgcctttgg agaaagtgga gtgtggcgct tggttgtcgt tatttcttcg gactgcttcg 60

cggtgcacgg attcagcttc tgcccagtgg ggctttcagc tgtttgcgcg tctctctgtc 120

cccctcccct ccccccggca cacctctgtc tacgatgagg aaaggtctgc gggcgacagc 180

ggcccgctgc ggactgggac tgggatactt gctgcaaatg ctcgtgctac ctgccctggc 240

cctgctcagc gccagcggca ctggctccgc cgcccaagat gatgactttt ttcatgaact 300

cccagaaact tttccttctg atccacctga gcctctgcca catttcctta ttgagcctga 360

agaagcttat attgtgaaga ataagcctgt gaacctgtac tgtaaagcaa gccctgccac 420

ccagatctat ttcaagtgta atagtgaatg ggttcatcag aaggaccaca tagtagatga 480

aagagtagat gaaacttccg gtctcattgt ccgggaagtg agcattgaga tttcgcgcca 540

gcaagtggaa gaactctttg gacctgaaga ttactggtgc cagtgtgtgg cctggagctc 600

cgcgggtacc acaaagagcc ggaaggcgta tgtgcgcatt gcatatctac ggaagacatt 660

tgagcaggaa cccctaggaa aggaagtgtc tttggaacag gaagtcttac tccagtgtcg 720

accacctgaa gggatcccag tggctgaggt ggaatggttg aaaaatgaag acataattga 780

tcccgttgaa gatcggaatt tttatattac tattgatcac aacctcatca taaagcaggc 840

ccgactctct gatactgcaa attacacctg tgttgccaaa aacattgttg ccaagaggaa 900

aagtacaact gccactgtca tagtctatgt caacggtggc tggtccacct ggacggagtg 960

gtctgtgtgt aacagccgct gtggacgagg gtatcagaaa cgtacaagga cttgtaccaa 1020

cccggcacca ctcaatgggg gtgccttctg tgaagggcag agtgtgcaga aaatagcctg 1080

tactacgtta tgcccagtgg atggcaggtg gacgccatgg agcaagtggt ctacttgtgg 1140

aactgagtgc acccactggc gcaggaggga gtgcacggcg ccagccccca agaatggagg 1200

caaggactgc gacggcctcg tcttgcaatc caagaactgc actgatgggc tttgcatgca 1260

gactgctcct gattcagatg atgttgctct ctatgttggg attgtgatag cagtgatcgt 1320

ttgcctggcg atctctgtag ttgtggcctt gtttgtgtat cggaagaatc atcgtgactt 1380

tgagtcagat attattgact cttcggcact caatgggggc tttcagcctg tgaacatcaa 1440

ggcagcaaga caagatctgc tggctgtacc cccagacctc acgtcagctg cagccatgta 1500

cagaggacct gtctatgccc tgcatgacgt ctcagacaaa atcccaatga ccaactctcc 1560

aattctggat ccactgccca acctgaaaat caaagtgtac aacacctcag gtgctgtcac 1620

cccccaagat gacctctctg agtttacgtc caagctgtcc cctcagatga cccagtcgtt 1680

gttggagaat gaagccctca gcctgaagaa ccagagtcta gcaaggcaga ctgatccatc 1740

ctgtaccgca tttggcagct tcaactcgct gggaggtcac cttattgttc ccaattcagg 1800

agtcagcttg ctgattcccg ctggggccat tccccaaggg agagtctacg aaatgtatgt 1860

gactgtacac aggaaagaaa ctatgaggcc acccatggat gactctcaga cacttttgac 1920

ccctgtggtg agctgtgggc ccccaggagc tctgctcacc cgccccgtcg tcctcactat 1980

gcatcactgc gcagacccca ataccgagga ctggaaaata ctgctcaaga accaggcagc 2040

acagggacag tgggaggatg tggtggtggt cggggaggaa aacttcacca ccccctgcta 2100

cattaagctg gatgcagagg cctgccacat cctcacagag aacctcagca cctacgccct 2160

ggtaggacat tccaccacca aagcggctgc aaagcgcctc aagctggcca tctttgggcc 2220

cctgtgctgc tcctcgctgg agtacagcat ccgagtctac tgtctggatg acacccagga 2280

tgccctgaag gaaattttac atcttgagag acagacggga ggacagctcc tagaagaacc 2340

taaggctctt cattttaaag gcagcaccca caacctgcgc ctgtcaattc acgatatcgc 2400

ccattccctc tggaagagca aattgctggc taaatatcag gaaattccat tttaccatgt 2460

ttggagtgga tctcaaagaa acctgcactg caccttcact ctggaaagat ttagcctgaa 2520

cacagtggag ctggtttgca aactctgtgt gcggcaggtg gaaggagaag ggcagatctt 2580

ccagctcaac tgcaccgtgt cagaggaacc tactggcatc gatttgccgc tgctggatcc 2640

tgcgaacacc atcaccacgg tcacggggcc cagtgctttc agcatccctc tccctatccg 2700

gcagaagctc tgtagcagcc tggatgcccc ccagacgaga ggccatgact ggaggatgct 2760

ggcccataag ctgaacctgg acaggtactt gaattacttt gccaccaaat ccagcccaac 2820

tggcgtaatc ctggatcttt gggaagcaca gaacttccca gatggaaacc tgagcatgct 2880

ggcagctgtc ttggaagaaa tgggaagaca tgaaacggtg gtgtccttag cagcagaagg 2940

gcagtattaa ccaccatgct ggaaggggaa atgaaggaca aaaatgcaca gggagtctgt 3000

ggccgtccag gtgaatcaca gctgaggagg aaatccagat gagaccaatg cacttcacag 3060

gcaagaatgc agcaggagcc agaaggaaaa cagatacaac tgcccatgta catgcccact 3120

ttactcggag atcatcacgg gagttaagaa aaattgtgta aatttgtacc ttgaatttag 3180

ctatcaacct aattttcctc ttagttgggc tgtatgctgt gtggtacagg atcttacagt 3240

ttcctaggaa acgcttttta ttgctatcca gatatatgga taaactttct taacaaaccc 3300

aatttctaca aatgttgttt acatcaaatt ggacagggat gcagacactg tccatggctc 3360

gttctatttt tgttcaaatc atttgaagtt gaagctgtgg acggtttgtt gtgtctattt 3420

cagattagta atttacagag aaatcacaga cttttgctac aaatcgtgtg catcaagtgt 3480

ctcagataat cctcccatca gtgttctgtt tctagaactt gtagaaccag tgttactgtt 3540

tgtatcaggg aagtggagaa tctaagtgta aaaaagaaat aactaagact cctattcctt 3600

ggagggaccc ttctggtgcc ctttgggaat aaagctgtag cactgc 3646

<210> 4
<211> 420
<212> DNA
<213> artificial sequence

<220>
<223> Region of the 1882_1883GC>AA mutation

<400> 4
caagggagag tctacgaaat gtatgtgact gtacacagga aagaaactat gaggccaccc 60

atggatgact ctcagacact tttgacccct gtggtgagct gtgggccccc aggagctctg 120

ctcacccgcc ccgtcgtcct cactatgcat cactgcgcag accccaatac cgaggactgg 180

aaaatactgc tcaagaacca gaaagcacag ggacagtggg aggatgtggt ggtggtcggg 240

gaggaaaact tcaccacccc ctgctacatt aagctggatg cagaggcctg ccacatcctc 300

acagagaacc tcagcaccta cgccctggta ggacattcca ccaccaaagc ggctgcaaag 360

cgcctcaagc tggccatctt tgggcccctg tgctgctcct cgctggagta cagcatccga 420

<210> 5
<211> 33
<212> DNA
<213> artificial sequence

<220>
<223> Primer U3A628KFor

<400> 5
cagctcaaaa accagaaagt gcagggacaa tgg 33

<210> 6
<211> 33
<212> DNA
<213> artificial sequence

<220>
<223> Primer U3A628KRev

<400> 6
ccattgtccc tgcactttct ggtttttgag ctg 33

<210>  7
<211>  24
<212>  DNA
<213>  artificial sequence

<220>
<223>  Left primer and probe specific to the region of the
       1882_1883GC>AA mutation

<400>  7
aatacaatgg aaatgtcact gaag                                              24


<210>  8
<211>  25
<212>  DNA
<213>  artificial sequence

<220>
<223>  Right primer and probe specific to the region of the
       1882_1883GC>AA mutation

<400>  8
ataaataaaa ggcctcccat aattg                                             25


<210>  9
<211>  932
<212>  PRT
<213>  artificial sequence

<220>
<223>  UNC5C sequence showing mutations P57T, R603C, A628K, A628E and
       Q630C

<400>  9

Met Arg Lys Gly Leu Arg Ala Thr Ala Ala Arg Cys Gly Leu Gly Leu
1               5                   10                  15


Gly Tyr Leu Leu Gln Met Leu Val Leu Pro Ala Leu Ala Leu Leu Ser
            20                  25                  30


Ala Ser Gly Thr Gly Ser Ala Ala Gln Asp Asp Asp Phe Phe His Glu
        35                  40                  45


Leu Pro Glu Thr Phe Pro Ser Asp Thr Pro Glu Pro Leu Pro His Phe
    50                  55                  60


Leu Ile Glu Pro Glu Glu Ala Tyr Ile Val Lys Asn Lys Pro Val Asn
65                  70                  75                  80


Leu Tyr Cys Lys Ala Ser Pro Ala Thr Gln Ile Tyr Phe Lys Cys Asn
                85                  90                  95

Ser Glu Trp Val His Gln Lys Asp His Ile Val Asp Glu Arg Val Asp
            100                 105                 110

Glu Thr Ser Gly Leu Ile Val Arg Glu Val Ser Ile Glu Ile Ser Arg
        115                 120                 125

Gln Gln Val Glu Glu Leu Phe Gly Pro Glu Asp Tyr Trp Cys Gln Cys
    130                 135                 140

Val Ala Trp Ser Ser Ala Gly Thr Thr Lys Ser Arg Lys Ala Tyr Val
145                 150                 155                 160

Arg Ile Ala Tyr Leu Arg Lys Thr Phe Glu Gln Glu Pro Leu Gly Lys
                165                 170                 175

Glu Val Ser Leu Glu Gln Glu Val Leu Leu Gln Cys Arg Pro Pro Glu
            180                 185                 190

Gly Ile Pro Val Ala Glu Val Glu Trp Leu Lys Asn Glu Asp Ile Ile
        195                 200                 205

Asp Pro Val Glu Asp Arg Asn Phe Tyr Ile Thr Ile Asp His Asn Leu
    210                 215                 220

Ile Ile Lys Gln Ala Arg Leu Ser Asp Thr Ala Asn Tyr Thr Cys Val
225                 230                 235                 240

Ala Lys Asn Ile Val Ala Lys Arg Lys Ser Thr Thr Ala Thr Val Ile
                245                 250                 255

Val Tyr Val Asn Gly Gly Trp Ser Thr Trp Thr Glu Trp Ser Val Cys
            260                 265                 270

Asn Ser Arg Cys Gly Arg Gly Tyr Gln Lys Arg Thr Arg Thr Cys Thr
        275                 280                 285

Asn Pro Ala Pro Leu Asn Gly Gly Ala Phe Cys Glu Gly Gln Ser Val
    290                 295                 300

Gln Lys Ile Ala Cys Thr Thr Leu Cys Pro Val Asp Gly Arg Trp Thr
305                 310                 315                 320

Pro Trp Ser Lys Trp Ser Thr Cys Gly Thr Glu Cys Thr His Trp Arg
                325                 330                 335

Arg Arg Glu Cys Thr Ala Pro Ala Pro Lys Asn Gly Gly Lys Asp Cys
            340                 345                 350

Asp Gly Leu Val Leu Gln Ser Lys Asn Cys Thr Asp Gly Leu Cys Met
        355                 360                 365

Gln Thr Ala Pro Asp Ser Asp Asp Val Ala Leu Tyr Val Gly Ile Val
    370                 375                 380

Ile Ala Val Ile Val Cys Leu Ala Ile Ser Val Val Val Ala Leu Phe
385                 390                 395                 400

Val Tyr Arg Lys Asn His Arg Asp Phe Glu Ser Asp Ile Ile Asp Ser
                405                 410                 415

Ser Ala Leu Asn Gly Gly Phe Gln Pro Val Asn Ile Lys Ala Ala Arg
            420                 425                 430

Gln Asp Leu Leu Ala Val Pro Pro Asp Leu Thr Ser Ala Ala Ala Met
        435                 440                 445

Tyr Arg Gly Pro Val Tyr Ala Leu His Asp Val Ser Asp Lys Ile Pro
    450                 455                 460

Met Thr Asn Ser Pro Ile Leu Asp Pro Leu Pro Asn Leu Lys Ile Lys
465                 470                 475                 480

Val Tyr Asn Thr Ser Gly Ala Val Thr Pro Gln Asp Asp Leu Ser Glu
                485                 490                 495

Phe Thr Ser Lys Leu Ser Pro Gln Met Thr Gln Ser Leu Leu Glu Asn
            500                 505                 510

Glu Ala Leu Ser Leu Lys Asn Gln Ser Leu Ala Arg Gln Thr Asp Pro
        515                 520                 525

Ser Cys Thr Ala Phe Gly Ser Phe Asn Ser Leu Gly Gly His Leu Ile
    530                 535                 540

Val Pro Asn Ser Gly Val Ser Leu Leu Ile Pro Ala Gly Ala Ile Pro
545                 550                 555                 560

Gln Gly Arg Val Tyr Glu Met Tyr Val Thr Val His Arg Lys Glu Thr
                565                 570                 575

Met Arg Pro Pro Met Asp Asp Ser Gln Thr Leu Leu Thr Pro Val Val
            580                 585                 590

Ser Cys Gly Pro Pro Gly Ala Leu Leu Thr Cys Pro Val Val Leu Thr
        595                 600                 605

Met His His Cys Ala Asp Pro Asn Thr Glu Asp Trp Lys Ile Leu Leu
    610                 615                 620

Lys Asn Gln Lys Glu Ala Cys Gly Gln Trp Glu Asp Val Val Val Val
625                 630                 635                 640

Gly Glu Glu Asn Phe Thr Thr Pro Cys Tyr Ile Lys Leu Asp Ala Glu
                645                 650                 655

Ala Cys His Ile Leu Thr Glu Asn Leu Ser Thr Tyr Ala Leu Val Gly
            660                 665                 670

His Ser Thr Thr Lys Ala Ala Ala Lys Arg Leu Lys Leu Ala Ile Phe
        675                 680                 685

Gly Pro Leu Cys Cys Ser Ser Leu Glu Tyr Ser Ile Arg Val Tyr Cys
    690                 695                 700

Leu Asp Asp Thr Gln Asp Ala Leu Lys Glu Ile Leu His Leu Glu Arg
705                 710                 715                 720

Gln Thr Gly Gly Gln Leu Leu Glu Glu Pro Lys Ala Leu His Phe Lys
                725                 730                 735

Gly Ser Thr His Asn Leu Arg Leu Ser Ile His Asp Ile Ala His Ser
            740                 745                 750

Leu Trp Lys Ser Lys Leu Leu Ala Lys Tyr Gln Glu Ile Pro Phe Tyr
        755                 760                 765

His Val Trp Ser Gly Ser Gln Arg Asn Leu His Cys Thr Phe Thr Leu
    770                 775                 780

Glu Arg Phe Ser Leu Asn Thr Val Glu Leu Val Cys Lys Leu Cys Val
785                 790                 795                 800

Arg Gln Val Glu Gly Glu Gly Gln Ile Phe Gln Leu Asn Cys Thr Val
                805                 810                 815

Ser Glu Glu Pro Thr Gly Ile Asp Leu Pro Leu Leu Asp Pro Ala Asn

820                 825                 830

Thr Ile Thr Thr Val Thr Gly Pro Ser Ala Phe Ser Ile Pro Leu Pro
        835                 840                 845

Ile Arg Gln Lys Leu Cys Ser Ser Leu Asp Ala Pro Gln Thr Arg Gly
    850                 855                 860

His Asp Trp Arg Met Leu Ala His Lys Leu Asn Leu Asp Arg Tyr Leu
865                 870                 875                 880

Asn Tyr Phe Ala Thr Lys Ser Ser Pro Thr Gly Val Ile Leu Asp Leu
                885                 890                 895

Trp Glu Ala Gln Asn Phe Pro Asp Gly Asn Leu Ser Met Leu Ala Ala
            900                 905                 910

Val Leu Glu Glu Met Gly Arg His Glu Thr Val Val Ser Leu Ala Ala
        915                 920                 925

Glu Gly Gln Tyr
    930

<210>  10
<211>  3749
<212>  DNA
<213>  homo sapiens

<220>
<221>  misc_feature
<223>  Homo sapiens UNC5 homolog A (UNC5A) - NCBI accession number
       NM_133369

<400>  10
gcattgctgc gctcccgtgc ccaagggagc cacgcgccgc gtgcgcccgg cagccggccg      60

cccggaggca gcgcagtccg ctggcatggg ccccgggggc gccccgagct ggggctccgg     120

gctgaggcgc taaagccgcc ctcccgcccg cggggccccg cgcccggccc gcccgcctgc     180

ccgcccgcgg ccatggccgt ccggcccggc ctgtggccag cgctcctggg catagtcctc     240

gccgcttggc tccgcggctc gggtgcccag cagagtgcca ccgtggccaa cccagtgcct     300

ggtgccaacc cggacctgct tccccacttc ctggtggagc ccgaggatgt gtacatcgtc     360

aagaacaagc cagtgctgct tgtgtgcaag gccgtgcccg ccacgcagat cttcttcaag     420

tgcaacgggg agtgggtgcg ccaggtggac cacgtgatcg agcgcagcac agacgggagc     480

agtgggctgc ccaccatgga ggtccgcatt aatgtctcaa ggcagcaggt cgagaaggtg     540

ttcgggctgg aggaatactg gtgccagtgc gtggcatgga gctcctcggg caccaccaag 600

agtcagaagg cctacatccg catagcctat ttgcgcaaga acttcgagca ggagccgctg 660

gccaaggagg tgtccctgga gcagggcatc gtgctgccct gccgtccacc ggagggcatc 720

cctccagccg aggtggagtg gctccggaac gaggacctgg tggacccgtc cctggacccc 780

aatgtataca tcacgcggga gcacagcctg gtggtgcgac aggcccgcct tgctgacacg 840

gccaactaca cctgcgtggc caagaacatc gtggcacgtc gccgcagcgc ctccgctgct 900

gtcatcgtct acgtggacgg cagctggagc ccgtggagca agtggtcggc ctgtgggctg 960

gactgcaccc actggcggag ccgtgagtgc tctgacccag caccccgcaa cggaggggag 1020

gagtgccagg gcactgacct ggacacccgc aactgtacca gtgacctctg tgtacacact 1080

gcttctggcc ctgaggacgt ggccctctat gtgggcctca tcgccgtggc cgtctgcctg 1140

gtcctgctgc tgcttgtcct catcctcgtt tattgccgga agaaggaggg gctggactca 1200

gatgtggctg actcgtccat tctcacctca ggcttccagc ccgtcagcat caagcccagc 1260

aaagcagaca acccccatct gctcaccatc cagccggacc tcagcaccac caccaccacc 1320

taccagggca gtctctgtcc ccggcaggat gggcccagcc ccaagttcca gctcaccaat 1380

gggcacctgc tcagccccct gggtggcggc cgccacacac tgcaccacag ctctcccacc 1440

tctgaggccg aggagttcgt ctcccgcctc tccacccaga actacttccg ctccctgccc 1500

cgaggcacca gcaacatgac ctatgggacc ttcaacttcc tcgggggccg gctgatgatc 1560

cctaatacag gaatcagcct cctcatcccc ccagatgcca taccccgagg gaagatctat 1620

gagatctacc tcacgctgca caagccggaa gacgtgaggt tgcccctagc tggctgtcag 1680

accctgctga gtcccatcgt tagctgtgga ccccctggcg tcctgctcac ccggccagtc 1740

atcctggcta tggaccactg tggggagccc agccctgaca gctggagcct gcgcctcaaa 1800

aagcagtcgt gcgagggcag ctgggaggat gtgctgcacc tgggcgagga ggcgccctcc 1860

cacctctact actgccagct ggaggccagt gcctgctacg tcttcaccga gcagctgggc 1920

cgctttgccc tggtgggaga ggccctcagc gtggctgccg ccaagcgcct caagctgctt 1980

ctgtttgcgc cggtggcctg cacctccctc gagtacaaca tccgggtcta ctgcctgcat 2040

gacacccacg atgcactcaa ggaggtggtg cagctggaga agcagctggg gggacagctg 2100

atccaggagc cacgggtcct gcacttcaag gacagttacc acaacctgcg cctatccatc 2160

cacgatgtgc ccagctccct gtggaagagt aagctccttg tcagctacca ggagatcccc 2220

ttttatcaca tctggaatgg cacgcagcgg tacttgcact gcaccttcac cctggagcgt 2280

gtcagcccca gcactagtga cctggcctgc aagctgtggg tgtggcaggt ggagggcgac 2340

gggcagagct tcagcatcaa cttcaacatc accaaggaca caaggtttgc tgagctgctg 2400

gctctggaga gtgaagcggg ggtcccagcc ctggtgggcc ccagtgcctt caagatcccc 2460

ttcctcattc ggcagaagat aatttccagc ctggacccac cctgtaggcg gggtgccgac 2520

tggcggactc tggcccagaa actccacctg gacagccatc tcagcttctt tgcctccaag 2580

cccagcccca cagccatgat cctcaacctg tgggaggcgc ggcacttccc caacggcaac 2640

ctcagccagc tggctgcagc agtggctgga ctgggccagc cagacgctgg cctcttcaca 2700

gtgtcggagg ctgagtgctg aggccggcca ggcccgacac ctacactctc accagctttg 2760

gcacccacca aggacaggca gaagccggac aggggccctt ccccacaccg gggagagctg 2820

ctcggacagg ccccctcccg gccgaagctg tcccttaatg ctggtccttc agaccctgcc 2880

cgaactccca cctctccatg gcctgcctag ccaggctggc actgccactc acactcggcc 2940

ccagggccca ggagggacag tgcctggagc ctgggccagg cccagcccat ctgtgtgtgt 3000

gtatgtgcgt gtgatgctac ctctcctccc gtccctctcc aggggccccg catacacacg 3060

gccatgcacg cacacactgg gcctgggcca gggccccaga gctcctgcct gagctggacc 3120

ttatgcaaac atttctgtgc ctgctgggta ggggcacgtc tgaggggccc tgctccaagc 3180

ctgcaggacc gagggccaca gccggacagg gggtagcccc tggattcagg cacacgacca 3240

ccacacgagc acgtgccacg catgcctcgt gtgctcatct cacacacacc cccctcccgg 3300

gtcacgcaga caccccccaa ccacacacat ctcatgctgt acacctgagg ctgctcacgt 3360

ctcacgccca gtgttggtgc acatttgcct ctcacatgct gccctctcca cccacccagg 3420

gacaccccac ggctcctccc tgcccctgcc cctcccccag ccttgaggtg ccctgcccgg 3480

cggggcctgt gaatatgcaa tgggagtccc aggctgtaca gtggtgagtg tgtgtgtggc 3540

gtggcgtgcc cgtccccagg gctggctggt gccccacgcg gggcctgtca tgtgaagctc 3600

gtgtcctgac tttgtcttaa gtgcattcac gcacttactc ttggccttat gtacacagcc 3660

ttgcccggcc gccggggcac ataggggttt tatcgggcgt gaatgtaaat aaattatata 3720

tatatattgc taaaaaaaaa aaaaaaaaa 3749

<210> 11
<211> 4998
<212> DNA
<213> homo sapiens

<220>
<221> misc_feature
<223> Homo sapiens UNC-5 homolog B (UNC5B) - NCBI accession number
NM_133369

<400> 11
actccggcga gcgagcggcg gcgctcggca ttgtggcagg cggctggggc cggctagggc 60

gccggagccg cacgcagccg cggggctccg agaggcgcgc actggggctg ggactgcgcg 120

gcgccgccgc tgcgagcgcc actgagcggt cgcgcaactt cggaggcaca gcgccggagc 180

caggcgagcg ctcagagacc cggagccaga ggggcgcgcc ggagcctcgt tcgagagccg 240

gcgccaggca cccaccgcgc tccgagtgcc aggcggccct ccgcgcagcg tggcttccgc 300

tgcccccacg gaaggcacgg gctggcgctg ccgggcgccg gggaggacgg cgaggaggag 360

gcggcggcgg cggagacggc ggcggcgaga ctggggccag ggagacagcc ctgggggaga 420

ggcgcccgaa ccaggccgcg ggagcatggg ggcccggagc ggagctcggg gcgcgctgct 480

gctggcactg ctgctctgct gggacccgag gctgagccaa gcaggcactg attctggcag 540

cgaggtgctc cctgactcct tcccgtcagc gccagcagag ccgctgccct acttcctgca 600

ggagccacag gacgcctaca ttgtgaagaa caagcctgtg gagctccgct gccgcgcctt 660

ccccgccaca cagatctact tcaagtgcaa cggcgagtgg gtcagccaga acgaccacgt 720

cacacaggaa ggcctggatg aggccaccgg cctgcgggtg cgcgaggtgc agatcgaggt 780

gtcgcggcag caggtggagg agctctttgg gctggaggat tactggtgcc agtgcgtggc 840

ctggagctcc gcgggcacca ccaagagtcg ccgagcctac gtccgcatcg cctacctgcg 900

caagaacttc gatcaggagc ctctgggcaa ggaggtgccc ctggaccatg aggttctcct 960

gcagtgccgc ccgccggagg gggtgcctgt ggccgaggtg gaatggctca agaatgagga 1020

tgtcatcgac cccacccagg acaccaactt cctgctcacc atcgaccaca acctcatcat 1080

ccgccaggcc cgcctgtcgg acactgccaa ctatacctgc gtggccaaga acatcgtggc 1140

caaacgccgg agcaccactg ccaccgtcat cgtctacgtg aatggcggct ggtccagctg 1200

ggcagagtgg tcaccctgct ccaaccgctg tggccgaggc tggcagaagc gcacccggac 1260

ctgcaccaac cccgctccac tcaacggagg ggccttctgc gagggccagg cattccagaa 1320

gaccgcctgc accaccatct gcccagtcga tggggcgtgg acggagtgga gcaagtggtc 1380

agcctgcagc actgagtgtg cccactggcg tagccgcgag tgcatggcgc ccccacccca 1440

gaacggaggc cgtgactgca gcgggacgct gctcgactct aagaactgca cagatgggct 1500

gtgcatgcaa aataagaaaa ctctaagcga ccccaacagc cacctgctgg aggcctcagg 1560

ggatgcggcg ctgtatgcgg ggctcgtggt ggccatcttc gtggtcgtgg caatcctcat 1620

ggcggtgggg gtggtggtgt accgccgcaa ctgccgtgac ttcgacacag acatcactga 1680

ctcatctgct gccctgactg gtggtttcca ccccgtcaac tttaagacgg caaggcccag 1740

caacccgcag ctcctacacc cctctgtgcc tcctgacctg acagccagcg ccggcatcta 1800

ccgcggaccc gtgtatgccc tgcaggactc caccgacaaa atccccatga ccaactctcc 1860

tctgctggac cccttaccca gccttaaggt caaggtctac agctccagca ccacgggctc 1920

tgggccaggc ctggcagatg gggctgacct gctgggggtc ttgccgcctg gcacataccc 1980

tagcgatttc gcccgggaca cccacttcct gcacctgcgc agcgccagcc tcggttccca 2040

gcagctcttg ggcctgcccc gagacccagg gagcagcgtc agcggcacct ttggctgcct 2100

gggtgggagg ctcagcatcc ccggcacagg ggtcagcttg ctggtgccca atggagccat 2160

tccccagggc aagttctacg agatgtatct actcatcaac aaggcagaaa gtaccctccc 2220

gctttcagaa gggacccaga cagtattgag cccctcggtg acctgtggac ccacaggcct 2280

cctgctgtgc cgccccgtca tcctcaccat gccccactgt gccgaagtca gtgcccgtga 2340

ctggatcttt cagctcaaga cccaggccca ccagggccac tgggaggagg tggtgaccct 2400

ggatgaggag accctgaaca caccctgcta ctgccagctg gagcccaggg cctgtcacat 2460

cctgctggac cagctgggca cctacgtgtt cacgggcgag tcctattccc gctcagcagt 2520

caagcggctc cagctggccg tcttcgcccc cgccctctgc acctccctgg agtacagcct 2580

ccgggtctac tgcctggagg acacgcctgt agcactgaag gaggtgctgg agctggagcg 2640

gactctgggc ggatacttgg tggaggagcc gaaaccgcta atgttcaagg acagttacca 2700

caacctgcgc ctctccctcc atgacctccc ccatgcccat tggaggagca agctgctggc 2760

caaataccag gagatcccct tctatcacat ttggagtggc agccagaagg ccctccactg 2820

cactttcacc ctggagaggc acagcttggc ctccacagag ctcacctgca agatctgcgt 2880

gcggcaagtg gaaggggagg gccagatatt ccagctgcat accactctgg cagagacacc 2940

tgctggctcc ctggacactc tctgctctgc ccctggcagc actgtcacca cccagctggg 3000

accttatgcc ttcaagatcc cactgtccat ccgccagaag atatgcaaca gcctagatgc 3060

ccccaactca cggggcaatg actggcggat gttagcacag aagctctcta tggaccggta 3120

cctgaattac tttgccacca aagcgagccc cacgggtgtg atcctggacc tctgggaagc 3180

tctgcagcag gacgatgggg acctcaacag cctggcgagt gccttggagg agatgggcaa 3240

gagtgagatg ctggtggctg tggccaccga cggggactgc tgagcctcct gggacagcgg 3300

gctggcaggg actggcagga ggcaggtgca gggaggcctg ggcagcctcc ctgatgggga 3360

tgtttggcct ctgcttcctc ccagttcaca gccagagttg cctctcctcc tcctcttccc 3420

caacccccag accatgacca gccttagaaa atccatgtac tctgttgtta gagggcccag 3480

agttccttct ccacccccgc tctctctctc ttggcctgag atctctgtgc aggaaccaag 3540

atggggctga agcctctgga ggcagttggt tggggcgggg caggcaggag gccctccctc 3600

caccccccca ccctcagccc ggcaacttct gggttccatg ggttttagtt ccgttctcgt 3660

tttcttcctc cgttattgat ttctcctttc tccctaagcc cccttctgct tccacgccct 3720

tttcctcttt gaagagtcaa gtacaattca gacaaactgc tttctcctgt ccaaaagcaa 3780

aaaggcaaag gaaagaaaga aagcttcaga ccgctagtaa ggctcaaaga agaagaaaaa 3840

caccaaaacc acaagggaaa agaaaaaccc agtttcttag gaaacgcaaa cgatttatta 3900

tccagattat ttggataagt cctttttaag aaaaaaaaag aaaatgaaaa acaacacaaa 3960

aaaaatagaa aactcttttc ttgagtgtgg atgagaatgg gcacgctcac acttctggcc 4020

aggagtgaat gtgcctgtgt gtgtgtgtgt gtgtgtgtgt gtgtgtgtac atgtacgcgt 4080

gtgcattgag gagagagaga ctgaacagag agtactttgc tgctaacgaa tccatcttgg 4140

actaaattct gacaaggcct cagtttcccc agttgtgcag ggagtaggtc agacagggtt 4200

ttcactgtct agatttagct ggttagattt tcctctaaaa tcttaagcag atgctgaatc 4260

cataaagcca aggagtgctg agggctggag tgggtgggcc ctccctccca cagccccagg 4320

gagatacatc tggactcctg ggaatctgga ttaagaaagt tgggatgaaa gaggaggact 4380

gctcaggcca gttgtgagcc agggagaatg ttccagtctg gttttggtct ggtggctcct 4440

agaaagggat gtggcccaga agtgggactc aaacagagag atgaagtagg ggaggggagt 4500

cctccacggg catctctctc ctggcggctg gatagcttta ccctagggtg gggccagccc 4560

tgacaccctc atcccctcct cagcccagca gcctctagag cagcgtggtg ctctcttgat 4620

ggccaggaat ccagaatctg cccttcccca accttggttt ctccttctgt aaagcagagt 4680

gatggctgag aagtcctctc tagctgcaga agcctgagct tcccggtgtc agtcagctct 4740

gcaacgtggg agccttggag gcgggtggcc tggctgtgct tcatatgccc aggagtggag 4800

aaggaggctg agaagctggt ttcccagctc tgatctccac aggcacctcc tataaccctc 4860

ctctcaccca ccccgctacg gtctgagaga tctgaaataa cctttcccag tgggcagggt 4920

tgccagggtt gaggggacag cacataccac ccccacccaa cctgttcgag gggccctgca 4980

tggcacggga tgagtccc 4998

<210> 12
<211> 2796
<212> DNA
<213> artificial sequence

<220>
<223> UNC5C variant showing mutations 169 C>A, 1807 C>T, 1888 C>G and
 c.1882_1883GC>AA

<400> 12
atgaggaaag gtctgcgggc gacagcggcc cgctgcggac tgggactggg atacttgctg 60

caaatgctcg tgctacctgc cctggccctg ctcagcgcca gcggcactgg ctccgccgcc 120

caagatgatg acttttttca tgaactccca gaaacttttc cttctgatac acctgagcct 180

ctgccacatt tccttattga gcctgaagaa gcttatattg tgaagaataa gcctgtgaac 240

ctgtactgta aagcaagccc tgccacccag atctatttca agtgtaatag tgaatgggtt 300

catcagaagg accacatagt agatgaaaga gtagatgaaa cttccggtct cattgtccgg 360

gaagtgagca ttgagatttc gcgccagcaa gtggaagaac tctttggacc tgaagattac 420

tggtgccagt gtgtggcctg gagctccgcg ggtaccacaa agagccggaa ggcgtatgtg 480

cgcattgcat atctacggaa gacatttgag caggaacccc taggaaagga agtgtctttg 540

gaacaggaag tcttactcca gtgtcgacca cctgaaggga tcccagtggc tgaggtggaa 600

tggttgaaaa atgaagacat aattgatccc gttgaagatc ggaattttta tattactatt 660

gatcacaacc tcatcataaa gcaggcccga ctctctgata ctgcaaatta cacctgtgtt 720

gccaaaaaca ttgttgccaa gaggaaaagt acaactgcca ctgtcatagt ctatgtcaac 780

ggtggctggt ccacctggac ggagtggtct gtgtgtaaca gccgctgtgg acgagggtat 840

cagaaacgta caaggacttg taccaacccg gcaccactca atgggggtgc cttctgtgaa 900

gggcagagtg tgcagaaaat agcctgtact acgttatgcc cagtggatgg caggtggacg 960

ccatggagca agtggtctac ttgtggaact gagtgcaccc actggcgcag gagggagtgc 1020

acggcgccag cccccaagaa tggaggcaag gactgcgacg gcctcgtctt gcaatccaag 1080

aactgcactg atgggctttg catgcagact gctcctgatt cagatgatgt tgctctctat 1140

gttgggattg tgatagcagt gatcgtttgc ctggcgatct ctgtagttgt ggccttgttt 1200

gtgtatcgga agaatcatcg tgactttgag tcagatatta ttgactcttc ggcactcaat 1260

gggggctttc agcctgtgaa catcaaggca gcaagacaag atctgctggc tgtaccccca 1320

gacctcacgt cagctgcagc catgtacaga ggacctgtct atgccctgca tgacgtctca 1380

gacaaaatcc caatgaccaa ctctccaatt ctggatccac tgcccaacct gaaaatcaaa 1440

gtgtacaaca cctcaggtgc tgtcaccccc caagatgacc tctctgagtt tacgtccaag 1500

ctgtcccctc agatgaccca gtcgttgttg gagaatgaag ccctcagcct gaagaaccag 1560

agtctagcaa ggcagactga tccatcctgt accgcatttg gcagcttcaa ctcgctggga 1620

ggtcacctta ttgttcccaa ttcaggagtc agcttgctga ttcccgctgg ggccattccc 1680

caagggagag tctacgaaat gtatgtgact gtacacagga aagaaactat gaggccaccc 1740

atggatgact ctcagacact tttgacccct gtggtgagct gtgggccccc aggagctctg 1800

ctcacctgcc ccgtcgtcct cactatgcat cactgcgcag accccaatac cgaggactgg 1860

aaaatactgc tcaagaacca gaaagcagag ggacagtggg aggatgtggt ggtggtcggg 1920

gaggaaaact tcaccacccc ctgctacatt aagctggatg cagaggcctg ccacatcctc 1980

acagagaacc tcagcaccta cgccctggta ggacattcca ccaccaaagc ggctgcaaag 2040

cgcctcaagc tggccatctt tgggcccctg tgctgctcct cgctggagta cagcatccga 2100

gtctactgtc tggatgacac ccaggatgcc ctgaaggaaa ttttacatct tgagagacag 2160

acgggaggac agctcctaga agaacctaag gctcttcatt ttaaaggcag cacccacaac 2220

ctgcgcctgt caattcacga tatcgcccat tccctctgga agagcaaatt gctggctaaa 2280

tatcaggaaa ttccatttta ccatgtttgg agtggatctc aaagaaacct gcactgcacc 2340

ttcactctgg aaagatttag cctgaacaca gtggagctgg tttgcaaact ctgtgtgcgg 2400

caggtggaag gagaagggca gatcttccag ctcaactgca ccgtgtcaga ggaacctact 2460

ggcatcgatt tgccgctgct ggatcctgcg aacaccatca ccacggtcac ggggcccagt 2520

gctttcagca tccctctccc tatccggcag aagctctgta gcagcctgga tgccccccag 2580

acgagaggcc atgactggag gatgctggcc cataagctga acctggacag gtacttgaat 2640

tactttgcca ccaaatccag cccaactggc gtaatcctgg atctttggga agcacagaac 2700

ttcccagatg gaaacctgag catgctggca gctgtcttgg aagaaatggg aagacatgaa 2760

acggtggtgt ccttagcagc agaagggcag tattaa 2796

<210> 13
<211> 20
<212> DNA
<213> Artificial

<220>
<223> Left primer and probe specific to the region of the
 1882_1883GC>AA mutation

<400> 13
acccaagtgc atagttcatg 20

<210> 14
<211> 20
<212> DNA
<213> Artificial

<220>
<223> Right primer and probe specific to the region of the
 1882_1883GC>AA mutation

<400> 14
tgctcctgct tatctgcatg 20

## Description

The present invention concerns the P57T, R603C, Q630C and A628K variants of the netrin-1 receptor UNC5C, said variants being implicated in the predisposition and carcinogenesis leading to solid cancers in human. The present invention relates to methods and materials used to isolate and detect a UNC5C gene, some mutant alleles of which cause susceptibility to cancer, in particular, colorectal cancer. More specifically, the invention relates to germline mutations in the UNC5C gene and their use in the diagnosis of predisposition to cancer. The present invention further relates to germinal and somatic mutations in the UNC5C gene in human colorectal cancer and their use in the diagnosis and prognosis of colorectal cancer. The detection of these UNC5C P57T, R603C, Q630C and A628K variants is useful in solid cancers and as a screening test for predisposition to solid cancers, such as colorectal cancers, in particular hereditary colorectal cancer syndrome as well as head and neck, thyroid, spinal cord, cerebral, pancreas, liver, abominal, bladder, ovary, and lung cancer. More generally, the invention is directed to a method for screening mutations in UNC5C resulting in loss of pro-apoptotic function which are implicated in carcinogenesis.

### Background of the invention

As of today, numerous genes have been identified as being directly linked to cancer risk when they are deleted or inactivated. Among these genes, they are well known tumour suppressor genes such as BRCA1, BRCA2, p53, CDKN2A and MMR and other genes that are recessive such as APC, CYLD, PTCH, VHL, WT1, and PTENRB. Another class comprises genes involved in DNA repair such as MUTYH, ATM, BLM, FANC, NBS1, RTS, WRN, and XP.

In addition, somes genes also linked to carcinogenesis are deleterious when over-activated or over-expressed. They genes are oncogenes such as the well known KIT, MET, PDGFRa and RET.

While these genes have been shown to be involved in various solid cancers, especially in familial cancers, research is on going to identify all potential risk genes. The ultimate goal is to provide screening and diagnostic tests that are the most exhautive. Beside, they are still many complex familial cancers that are under evalutions as key predisposing genes remains elusive. Therefore, in such complex familial cancer cases, the problem is to idenfify the key genes and key mutations thereof that are responsible for the predisposition to cancer and tumorogenesis.

Once a risk gene is identified, it is possible to screen whithin the sequence which mutations are the most prevalent in the general population or in subpopulations afflicted with higher risk of cancer. This in turn allows to design reliable screening tests or diagnostic tests.

For example, most familial colorectal cancers are believed to originate from mutations in specific sets of tumor suppressor genes or oncogenes but most of them are, so far, unknown. Indeed, while high-penetrance germline mutations in known genes explain less that 10% of colorectal cancer cases (1), recent studies suggest that heritability accounts for 35% of the variation in susceptibility to this cancer. Much of the remaining variation in genetic risk is then probably due to mutations in unknown susceptibility genes, each exerting an influence on the development of colorectal cancer.

We report here the identification of a new risk gene responsible for familial colorectal cancers and others cancers. We further discovered a common germline A628K mutation in the UNC5C gene, probably emerging from a common founder that affects the pro-apoptotic activity of this receptor and which segregates with colorectal cancer in families with complex cancer history. We also found several other common germline mutations, namely P57T, R603C, Q630C. Taken together, these results allow to design diagnostic tools useful to screen the general population and more specifically populations at risk including members of kindred in which a first case displaying the above variants is reported.

The netrin-1 receptor UNC5C belongs to the functional family of dependence receptors (6) including UNC5A and UNCB (also referred as UNC5H genes - UNC5H1-3). The pro-apoptotic activity of these receptors observed in the absence of their respective ligand has been hypothesized to confer a tumour suppressor activity to these receptors. Indeed, expression of one of these dependence receptors at the surface of a tumour cell is thought to render cell survival dependent on ligand availability, thereby inhibiting uncontrolled cell proliferation or metastasis (7). We and others have recently demonstrated that UNC5C behaves as a tumour suppressor: (i) it is down-regulated through promoter methylation in a large fraction of human sporadic colorectal cancers (CRCs) (4) (5); and (ii) UNC5C inactivation in mice increases intestinal tumor progression when associated with the APC (adenomatous polyposis coli) predisposing mutation (4).

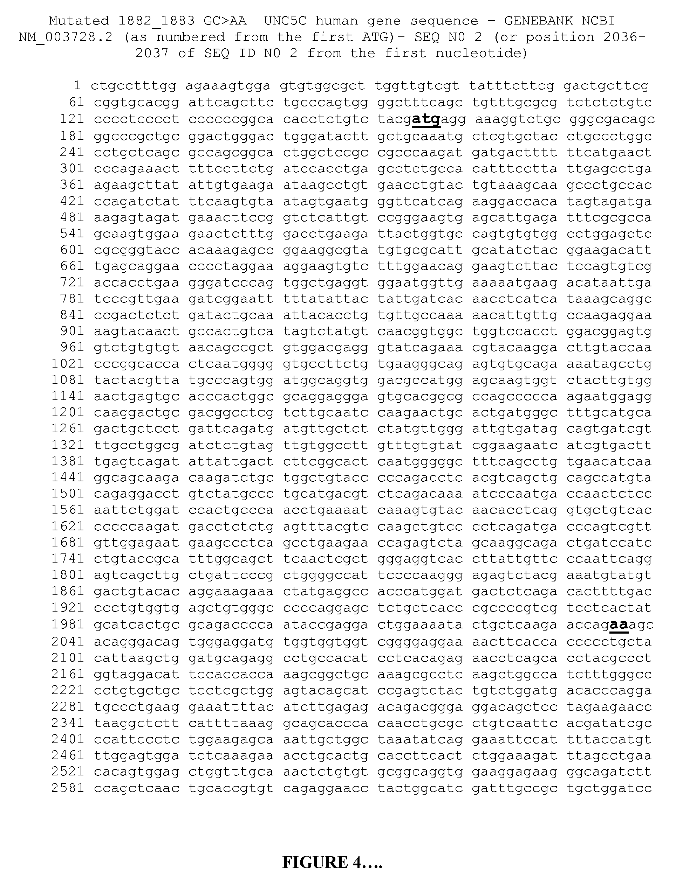

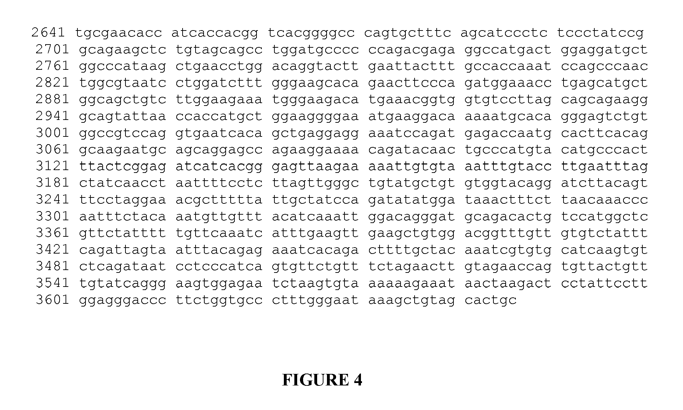

We show here that UNC5C genetic abnormalities is involved in human colorectal carcinogenesis and we provide the identification of germline mutations in the human UNC5C gene that increases predisposition to cancer. This invention allows the screening of deleterious mutations in UNC5C to predict cancer risk and provides a diagnostic test to detect the UNC5C A628K variant and other variants in patients and their relatives, in particular in patients afflicted with colorectal cancer, as well as head and neck, thyroid, spinal cord, cerebral, pancreas, liver, blader, ovary and lung cancer. Finally, the detection of an HNPCC-associated mutation (hereditary nonpolyposis colorectal cancer) in the UNC5C gene calls for a larger screen for HNPCC-associated mutations in netrin-1 dependence receptors-induced cell death pathways. Along this line, we detected a missense mutation (V5031) in the ZU5 domain of UNC5A gene in two patients of a family diagnosed with HNPCC (Supplementary Fig.4).

### DESCRIPTION OF THE INVENTION

### 1 - Sequences

Therefore, according to a first embodiment, the present invention concerns the isolated protein UNC5C (unc-5 homolog C), in particular the Homo sapiens UNC5C (NCBI, accession number NM_003728.2 GI:16933524) comprising a mutation on amino acid 628 (codon 628 of the cDNA starting from ATG), more particularly the A628K mutation, hereinafter called UNC5C A628K variant such as presented in SEQ ID No 1 herebelow :

### SEQ ID No 1 - Mutated UNC5C A628K

MRKGLRATAARCGLGLGYLLQMLVLPALALLSASGTGSAAQDDDFFHEL
PETFPSDPPEPLPHFLIEPEEAYIVKNKPVNLYCKASPATQIYFKCNSEWV
HQKDHIVDERVDETSGLIVREVSIEISRQQVEELFGPEDYWCQCVAWSS
AGTTKSRKAYVRIAYLRKTFEQEPLGKEVSLEQEVLLQCRPPEGIPVAEV

EWLKNEDIIDPVEDRNFYITIDHNLIIKQARLSDTANYTCVAKNIVAKRKST
TATVIVYVNGGWSTWTEWSVCNSRCGRGYQKRTRTCTNPAPLNGGAF
CEGQSVQKIACTTLCPVDGRWTPWSKWSTCGTECTHWRRRECTAPAP
KNGGKDCDGLVLQSKNCTDGLCMQTAPDSDDVALYVGIVIAVIVCLAISV
VVALFVYRKNHRDFESDIIDSSALNGGFQPVNIKAARQDLLAVPPDLTSA
AAMYRGPVYALHDVSDKIPMTNSPILDPLPNLKIKVYNTSGAVTPQDDLS
EFTSKLSPQMTQSLLENEALSLKNQSLARQTDPSCTAFGSFNSLGGHLIV
PNSGVSLLIPAGAIPQGRVYEMYVTVHRKETMRPPMDDSQTLLTPVVSC
GPPGALLTRPVVLTMHHCADPNTEDWKILLKNQK628AQGQWEDVVVV
GEENFTTPCYIKLDAEACHILTENLSTYALVGHSTTKAAAKRLKLAIFGPL
CCSSLEYSIRVYCLDDTQDALKEILHLERQTGGQLLEEPKALHFKGSTHN
LRLSIHDIAHSLWKSKLLAKYQEIPFYHVWSGSQRNLHCTFTLERFSLNT
VELVCKLCVRQVEGEGQIFQLNCTVSEEPTGIDLPLLDPANTITTVTGPSA
FSIPLPIRQKLCSSLDAPQTRGHDWRMLAHKLNLDRYLNYFATKSSPTGV
ILDLWEAQNFPDGNLSMLAAVLEEMGRHETVVSLAAEGQY

The invention also concerns similar proteins bearing the mutated K at position 628 in other mammals, and variants of SEQ ID No 1 which also comprise one or more simple polymorphisms, called polymorphic variant, which do not affect the activity and 3D structure of the protein.

Such UNC5C A628K variants are useful to screen candidate compounds or as target for specific monoclonal antibodies.

The invention also relates to variants of the UNC5C protein which results in loss of the pro-apoptotic function, such as a variant of UNC5C comprising one of the following mutations : P57T, R603C, A628K, A628E, and Q630C as shown below (SEQ ID No 9):

MRKGLRATAARCGLGLGYLLQMLVLPALALLSASGTGSAAQDDDFFHEL
PETFPSDT57PEPLPHFLIEPEEAYIVKNKPVNLYCKASPATQIYFKCNSE
WVHQKDHIVDERVDETSGLIVREVSIEISRQQVEELFGPEDYWCQCVAW

SSAGTTKSRKAYVRIAYLRKTFEQEPLGKEVSLEQEVLLQCRPPEGIPVA
EVEWLKNEDIIDPVEDRNFYITIDHNLIIKQARLSDTANYTCVAKNIVAKRK
STTATVIVYVNGGWSTWTEWSVCNSRCGRGYQKRTRTCTNPAPLNGG
AFCEGQSVQKIACTTLCPVDGRWTPWSKWSTCGTECTHWRRRECTAP
APKNGGKDCDGLVLQSKNCTDGLCMQTAPDSDDVALYVGIVIAVIVCLAI
SVVVALFVYRKNHRDFESDIIDSSALNGGFQPVNIKAARQDLLAVPPDLT
SAAAMYRGPVYALHDVSDKIPMTNSPILDPLPNLKIKVYNTSGAVTPQDD
LSEFTSKLSPQMTQSLLENEALSLKNQSLARQTDPSCTAFGSFNSLGGH
LIVPNSGVSLLIPAGAIPQGRVYEMYVTVHRKETMRPPMDDSQTLLTPVV
SCGPPGALLTC603PVVLTMHHCADPNTEDWKILLKNQK/E628AC630G
QWEDVVVVGEENFTTPCYIKLDAEACHILTENLSTYALVGHSTTKAAAKR
LKLAIFGPLCCSSLEYSIRVYCLDDTQDALKEILHLERQTGGQLLEEPKAL
HFKGSTHNLRLSIHDIAHSLWKSKLLAKYQEIPFYHVWSGSQRNLHCTFT
LERFSLNTVELVCKLCVRQVEGEGQIFQLNCTVSEEPTGIDLPLLDPANTI
TTVTGPSAFSIPLPIRQKLCSSLDAPQTRGHDWRMLAHKLNLDRYLNYFA
TKSSPTGVILDLWEAQNFPDGNLSMLAAVLEEMGRHETVVSLAAEGQY

The invention also relates to a nucleotide sequence encoding a UNC5C variant comprising a mutation as shown in SEQ ID No 9 above. For example, it may be a sequence comprising one or more mutations selected from 169 C>A , 1807 C>T, 1888 C>G and c.1882_1883GC>AA as shown in SEQ ID No 12. P57T corresponds to 169 C>A, Q630C corresponds to 1888 C>G and R603C corresponds to 1807 C>T.

More specifically, the invention relates to a nucleotide sequence encoding SEQ ID No 1, preferably SEQ ID No 2 (sequence of the human gene with the AAA codon instead of GCA in codon 628 (a G>A transition followed by a C>A transversion (c.1882_1883GC>AA) in exon 11 that causes the amino acid change p.Ala628Lys (A628K) ; positions 1882 and 1883 are calculated from the ATG marking the start of translation).

The above nucleotide sequences may be found in a viral or plasmid vector, or a naked DNA under the control of a efficient promoter in mammalian cells. The invention therefore extends to a vector expressing the above variants of UNC5C, in particular the UNC5C A628K protein.

The vector of the invention may be a cloning and/or expression vector and may be used to transfect a host cell, in particular a mammalian cell, such as for example a non human embryonnic stem cell, to allow to generate mice or rat models for example.

### 2 - Model Transgenic Animal of colorectal cancer and other solid cancer involving the UNC5C A628K protein :

The invention also concerns a non-human transgenic animal expressing recombinant UNC5C A628K protein. This animal may preferably be a mouse or rat. Transgenic rats or mice which can be used as models may be obtained by any method commonly used by those skilled in the art, in particular by a Knock-in method (targeted insertion of a sequence) by homologous recombination or directed recombination with the Cre-LoxP or FLP-FRT systems in ES cells. According to one preferred embodiment of the invention, the transgenic cell is obtained by gene targeting of the UNC5C A628K sequence at one or more sequences of the host cell genome. More precisely, the transgene is inserted stable fashion by homologous recombination at the homologous sequences in the genome of the host cell. When it is desired to obtain a transgenic cell with a view to producing a transgenic animal, the host cell is preferably an embryonic stem cell (ES cell) (Thompson et al, 1989). Gene targeting is the directed modification of a chromosome locus by homologous recombination with an exogenous DNA sequence having sequence homology with the targeted endogenous sequence. There are different types of gene targeting. Here, gene targeting may be used more particularly to replace the wild-type UNC5C gene by the gene variant UNC5C A628K or any other genetically similar variant. In this case, the gene targeting is called "Knock-in" (K-in).

Alternatively, gene targeting may be used to reduce or annihilate expression of wild-type UNC5C. This is then called "Knock-out" gene targeting (KO) (see Bolkey et al, 1989). The cell of the invention is **characterized in that** the transgene is integrated stably into the genome of said cell, and in that its expression is controlled by the regulatory elements of the endogenous gene. By stable integration, it is meant the insertion of the transgene into the genomic DNA of the inventive cell. The transgene so inserted is then transmitted to cell progeny. Integration of the transgene is made upstream, downstream or in the centre of the target UNC5C endogenous gene. Optionally, one or more positive or negative selection genes may be used. It is also possible to use DNA homology regions with the target locus, preferably a total of two, located either side of the reporter gene portion or either side of the complete sequence to be inserted. By "DNA homology regions" is meant two DNA sequences which, after optimal alignment and after comparison, are identical for usually at least approximately 90% to 95% of the nucleotides and preferably at least 98 to 99.5% of the nucleotides. Optimal alignment of the sequences for comparison may be made using the Smith-Waterman local homology algorithm (1981), the Neddleman-Wunsch local homology algorithm (1970), the similarity search method of Pearson and Lipman (1988), or computer software using these algorithms (GAP, BESTFIT, BLAST P, BLAST N, FASTA and TFASTA in the Wisconsin Genetics Software Package, Genetics Computer Group, 575 Science Dr., Madison, Wis.). Although as few as 14 bp with 100% homology are sufficient to achieve homologous recombination in bacteria and mammalian cells, longer portions of homologous sequences are preferred (in general the size of these portions is at least 2000 bp, preferably at least 5000 bp for each portion of homologous sequence. Advantageously, the UNC5C variant sequence is inserted in the group of elements ensuring endogenous type regulation, i.e. a group comprising at least the promoter, regulator sequences (enhancers, silencers, insulators) and the terminating signals of the endogenous UNC5C gene.

According to one particular embodiment, the transgene UNC5C A628K comprises at least the coding sequence, a positive selection cassette whether flanked or not by sites specific to the action of the recombinases, e.g. a Lox/Neo-TK/Lox cassette or lox/Neo/lox or FRT/Neo-TK/FRT ou FRT/Neo/FRT cassette possibly also being present at position 5' of said sequence, and **characterized in that** a negative selection cassette for example containing the DTA and/or TK gene or genes is at least present at one of the ends of the transgene. The transgene of the present invention is preferably directly derived from an exogenous DNA sequence naturally present in an animal cell. This DNA sequence in native form may be altered for example through the insertion of restriction sites needed for cloning and/or through the insertion of site-specific recombination sites (1ox and flp sequences).

For this purpose, the UNC5C A628K variant can be cloned in a cloning vector ensuring its propagation in a host cell, and/or optionally in an expression vector to ensure expression of the transgene. The recombinant DNA technologies used to construct the cloning and/or expression vector of the invention are known to those skilled in the art. Standard techniques are used for cloning, DNA isolation, amplification and purification; enzymatic reactions involving DNA ligase, DNA polymerase, restriction endonucleases are performed following the manufacturer's instructions. These techniques, and others are generally conducted in accordance with Sambrook et al, 1989. The vectors include plasmids, cosmids, bacteriophages, retroviruses and other animal viruses, artificial chromosomes such as YAC, BAC, HAC and other similar vectors.

The methods for generating transgenic cells of the invention are described in Gordon et al, 1989. Various techniques for transfecting mammalian cells were reviewed by Keon et al, 1990. The inventive transgene, optionally contained in a linearised or non-linearised vector, or in the form of a vector fragment, can be inserted in the host cell using standard methods such as microinjection into the nucleus for example (U.S. Pat. No. 4,873,191), transfection by calcium phosphate precipitation, lipofection, electroporation (Lo, 1983), heat shock, transformation with cationic polymers (PEG, polybrene, DEAE-Dextran.) or viral infection (Van der Putten et al, 1985).

When the cells have been transformed by the transgene, they may be cultured in vitro or else used to produce non-human transgenic animals. After transformation, the cells are seeded on a nutritional layer and/or in a suitable medium. The cells containing the construct can be detected using a selective medium. After a sufficient time to allow the colonies to grow, they are then collected and analysed to determine whether or not a homologous recombinant event and/or integration of the construct has occurred. To screen the clones possibly fulfilling homologous recombination, positive and negative markers, also called selection genes, may be inserted in the homologous recombination vector. Different systems for selecting cells producing the homologous recombination event have been described (for review U.S. Pat. No. 5,627,059). Said positive selection gene of the invention is preferably chosen from among antibioticresistant genes. Among the antibiotics a non-exhaustive list comprises neomycin, tetracycline, ampicilline kanamycin, phleomycin, bleomycin, hygromycin, chloramphenicol, carbenicilline, geneticine, puromycin. The resistance genes corresponding to these antibiotics are known to those skilled in the art; as an example the resistance gene to neomycin makes the cells resistant to the presence of the G418 antibiotic in the culture medium. The positive selection gene may also be chosen from among the HisD gene, the corresponding selective agent being histidinol. The positive selection gene may also be chosen from among the gene of guanine-phosphoribosyl-transferase (GpT), the corresponding selective agent being xanthine. The positive selection gene may also be chosen from among the hypoxanthine-phosphoribosyl-transferase gene (HPRT), the corresponding selective agent being hypoxanthine. The selection marker or markers used to allow identification of homologous recombination events may subsequently affect gene expression, and may be removed if necessary using specific site recombinases such as the Cre recombinase specific to Lox sites (Sauer, 1994; Rajewsky et al, 1996; Sauer, 1998) or FLP specific to FRT sites (Kilby et al, 1993).

The positive colonies, i.e. containing cells in which at least one homologous recombinant event has occurred, are identified by Southern blotting analysis and/or PCR techniques. The expression level, in the isolated cells or cells of the inventive transgenic animal, of the mRNA corresponding to the transgene may also be determined by techniques including Northern blotting analysis, in situ hybridisation analysis, by RT-PCR. Also, the cells or animal tissues expressing the transgene may be identified using an antibody directed against the reporter protein. The positive cells may then be used to conduct embryo handling procedures in particular the injection of cells modified by homologous recombination into the blastocysts.

Regarding mice, the blastocysts are obtained from 4 to 6-week superovulated females. The cells are trypsinated and the modified cells are injected into the blastocele of a blastocyst. After injection, the blastocysts are inserted into the uterine horn of pseudo-pregnant females. The females are then allowed to reach full term and the resulting offspring are analysed to determine the presence of mutant cells containing the construct. Analysis of a different phenotype between the cells of the newborn embryo and the cells of the blastocyst or ES cells makes it possible to detect chimeric newborn. The chimeric embryos are then raised to adult age. The chimera or chimeric animals are animals in which only a sub-population of cells contains an altered genome. Chimeric animals having the modified gene or genes are generally cross-bred between each other or with a wild-type animal to obtain either heterozygous or homozygous offspring. Male and female heterozygotes are then cross-bred to generate homozygous animals. Unless otherwise indicated, the non-human transgenic animal of the invention comprises stable changes in the nucleotide sequence of germ line cells.

According to another embodiment of the invention, the non-human transgenic cell may be used as nucleus donor cell for the transfer of a nucleus, or nuclear transfer. By nuclear transfer, it is meant the transfer of a nucleus from a living donor cell of a vertebrate, an adult or foetal organism, into the cytoplasm of an enucleated receiver cell of the same species or a different species. The transferred nucleus is reprogrammed to direct the development of cloned embryos which can then be transferred to foster females to produce the foetuses and newborn, or can be used to produce cells of the inner cell mass in culture. Different nuclear cloning techniques may be used; among these, non-exhaustive mention may be made of those subject of patent applications WO 95 17500, WO 97/07668, WO 97 07669, WO 98 30683, WO 99 01163 and WO 99 37143.

Therefore, the invention also extends to a non-human transgenic animal comprising a recombinant sequence encoding the variant UNC5C A628K. These animals may be homozygous or heterozygous (UNC5C A628K / UNC5C A628K or UNC5C A628K / UNC5C). In particular, these animals reproduce susceptibility to colorectal cancers but also any other solid cancers in which carcinogenesis involves inactivation of UNC5C. These models can therefore be used to conduct functional screening of antitumoral compounds or compounds which restores the function of UNC5C as tumor suppressor gene.

Another embodiement consists of injecting a viral vector for gene therapy able to express UNC5C to restore UNC5C activity in sub-population of patients that are homozygous or heterozygous UNC5C A628K / UNC5C A628K or UNC5C A628K / UNC5C.

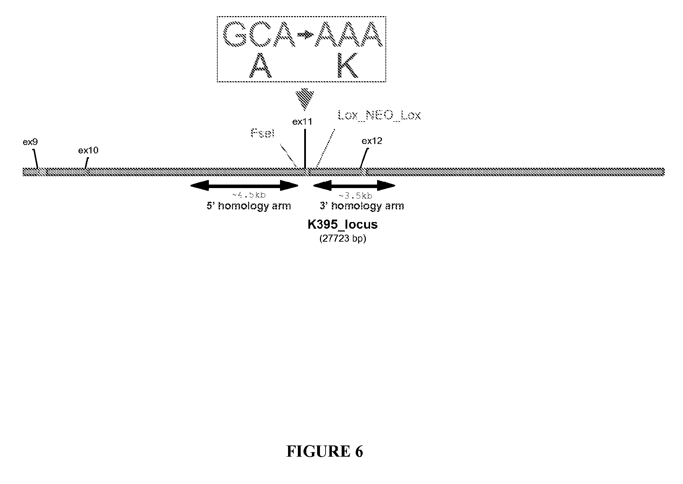

In one particular embodiment, the UNC5C A628K mouse is as described in Figures 6.

### 3 - Screening and Diagnostic Tools

The invention relates to screening and diagnostic tools to detect the presence or absence of variants in the UNC5C gene resulting in loss of pro-apoptotic function, especially variants selected from P57T, R603C, Q630C A841Y, A628E and A628K or any other alterations in the UNC5C gene in mammals, especially in human, suffering from or likely to show a solid cancer, especially colorectal cancers, head and neck, thyroid, spinal cord, pancreas, liver, or lung cancer.

Alterations of the wild-type UNC5C gene are detected. The method can be performed by detecting the wild-type UNC5C gene and confirming the lack of a predisposition to cancer. On the other side, the method can be performed by detecting the alterations in the UNC5C gene and confirming a predisposition to cancer.

"Alteration of a wild-type gene" encompasses all forms of mutations including deletions, insertions and point mutations in the coding and noncoding regions. Deletions may be of the entire gene or of only a portion of the gene. Point mutations may result in stop codons, frameshift mutations or amino acid substitutions. Somatic mutations are those which occur only in certain tissues, such as in the tumor tissue, and are not inherited in the germline. Germline mutations can be found in any of a body's tissues and are inherited. Thus it is an object of the invention to screen for mutations, such as insertions, small deletions, and point mutations. It is believed that many mutations found in tumor tissues will be those leading to decreased expression or inactivation of the UNC5C gene product. Point mutational events may occur in regulatory regions, such as in the promoter of the gene, leading to loss or diminution of expression of the mRNA.Point mutations may also abolish proper RNA processing, leading to loss of expression of the UNC5C gene product, or to a decrease in mRNA stability or translation efficiency.

Among alterations to screen, the method according to the invention includes the A841Y, any alteration in position 628 including A628E as well as P57T, R603C, and Q630C. For P57T, we have discovered that this mutation is particuarly associated with solid cancers such as liver and bladder cancer and abdominal carcinoma. For R603C, we have discovered that this mutation is particuarly associated with solid cancers such as ovary cancer, cerabral tumors and also with blood cancer such as CML (chronic myeloid leukemia). For Q630E, we have discovered that this mutation is particuarly associated with solid cancers such as lung cancer. Considering the general implication of alterations of UNC5C in various cancers, the aim of the present invention is to screen patients unlimited to the underlying, suspected, or risk to develop a proliferative disorder. Thus, the invention is directed to a method for screening alterations which comprises determining whether there is an alteration in the sequence of the UNC5C gene in a tissue sample of said subject compared to the nucleotide sequence of wild type UNC5C gene set forth in SEQ ID No 3 or a wild-tyle allelic variant thereof, said alteration indicating a predisposition to said cancer.

In one particlular embodiment, the invention relates to an in vitro method to determine if a human patient is currently suffering from or is likely to develop a solid cancer, such as colorectal cancers, head and neck, thyroid, spinal cord, pancreas, liver, and lung cancer, comprising:
a) analyzing a nucleic acid sample from the human patient;
b) detecting the presence or absence of variants in the UNC5C gene (human unc-5 homolog C, the wild-type sequence of which being displayed as SEQ ID No 3 or polymorphic variant thereof), wherein said variants results in the loss of the pro-apoptotic function of the UNC5C protein and wherein the presence of said variant in said UNC5C gene indicates that the human patient is currently suffering from or is likely to develop solid cancer. This method comprises the detection of at least one variant selected from P57T, R603C, Q630C A841Y, A628E and A628K in the UNC5C gene as shown in SEQ ID No 9.

More specifically, the invention is directed to a screening test to detect deleterious mutations or alterations as decribed above that are deletions, insertion or point mutations, especially mutations resulting in frameshift or resulting in the loss of expression or activity of the UNC5C gene product. This method may be practiced by a number of ways known to the skilled person, for example fluorescent in situ hybridization (FISH), direct DNA sequencing, PFGE analysis, Southern blot analysis, single stranded conformation analysis (SSCA), RNase protection assay, allele-specific oligonucleotide (ASO), dot blot analysis and PCR-SSCP.

Direct DNA sequencing such as automated fluorescent sequencing can detect sequence variation. Another approach is the single-stranded conformation polymorphism assay (SSCA) (Orita et al., 1989). This method does not detect all sequence changes but can be optimized to detect most DNA sequence variation. The fragments which have shifted mobility on SSCA gels are then sequenced to determine the exact nature of the DNA sequence variation. Once a mutation is known, an allele specific detection approach such as allele specific oligonucleotide (ASO) hybridization can be utilized to rapidly screen large numbers of other samples for that same mutation.

Screening or detection of known mutation according to the above may also be performed by treating nucleic acid samples with endonuclease-1 (endo-1) (for example from Serial Genetics) and detection heteroduplexes. In this regard, PCR amplification products are denaturated at 95°C, then slowly renaturated with gradual decrease of 1°C per min until reaching 5°C. This allows the formation of heteroduplexes between two amplified strands. Mismatches form and are detected by endo-1 which cleaves the partially hybridized strands at the mismatches (Triques K, Piednoir E, Dalmais M, et al. Mutation detection using ENDO1: application to disease diagnostics in humans and TILLING and Eco-TILLING in plants. BMC Mol Biol. 2008;9:42 ; incorporated herein by reference). Digested PCR fragments are then run on 3% agarose gels and samples displaying bands generated by cleavage are then sequenced to detect or identify mutations.

The invention also relates to a method for diagnosing a predisposition for solid cancers such as colorectal cancers, in particular hereditary colorectal cancer syndrome as well as head and neck, thyroid, spinal cord, pancreas, liver, ovary, bladder, cerebral, abdominal and lung cancer in a human subject which comprises determining whether there is a germline alteration in the sequence of the UNC5C gene in a tissue sample of said subject compared to the nucleotide sequence set forth in SEQ ID No 3 or a wild-tyle allelic variant thereof, said alteration indicating a predisposition to said cancer.

In particular, the mutation is selected from the 1882_1883GC>AA as show in SEQ ID No 2.

In this respect, the invention relates to primers and probes with which to detect the presence or absence of the mutation in the SEQ ID No 2 sequence described above. More particularly, the invention embraces an isolated nucleic acid having a sequence of at least 10, 12, 15, 20, 30, 40 or 50 consecutive nucleotides (e.g. 10 to 30 nucleotides or 10 to 25 nucleotides) of exon 11 or of the sequence SEQ ID No 2 in the region of the 1882_1883GC>AA mutation including or not the mutated nucleotides at position 1882 and 1883 refering to the first ATG marking the initiation of translation. One region of interest is shown below as SEQ ID No 4.

The underlined sequence designates an example of upstream or downstream areas allowing the design of probes or primers specific to the mutation at position the 1882_1883GC>AA mutation :

Example of Different Preferred Primers and Probes of the Invention :

SEQ ID No 4
1681 caagggagag tctacgaaat gtatgtgact gtacacagga aagaaactat
gaggccaccc
1741 atggatgact ctcagacact tttgacccct gtggtgagct gtgggccccc aggagctctg
1801 ctcacccgcc ccgtcgtcct cactatgcat cactgcgcag accccaatac cgaggactgg
1861 aaaatactgc tcaagaacca gaaagcacag ggacagtggg aggatgtggt
ggtggtcggg

1921 gaggaaaact tcaccacccc ctgctacatt aagctggatg cagaggcctg ccacatcctc
1981 acagagaacc tcagcaccta cgccctggta ggacattcca ccaccaaagc
ggctgcaaag
2041 cgcctcaagc tggccatctt tgggcccctg tgctgctcct cgctggagta cagcatccga

Examples of prefered primers and probes include SEQ ID No 7, 8, 13 and 14.

| | |
|---|---|
| left | right |
| AATACAATGGAAATGTCACTGAAG | ATAAATAAAAGGCCTCCCATAATTG |
| SEQ ID No 7 | SEQ ID No 8 |
| ACCCAAGTGCATAGTTCATG | TGCTCCTGCTTATCTGCATG |
| SEQ ID No 13 | SEQ ID No 14 |

The presence of absence of a nucleic acid comprising the 1882_1883GC>AA mutation corresponding to the UNC5C A628K variant can be detected by sequencing, amplification and/or hybridisation with a specific probe and specific primers such as sequence derived from SEQ ID No 2 or 4 as described.

The invention therefore provides an in vitro method to determine the presence or absence of the 1882_1883 GC>AA UNC5C mutation in the UNC5C gene (human unc-5 homolog C, the wild-type sequence of which being displayed as SEQ ID No 3 or polypohormic variant thereof), which mutation results in the UNC5C A628K variant protein of SEQ ID No 1, in a human patient comprising:
a) analyzing a nucleic acid sample from the human patient;
b) detecting a AA in the UNC5C gene at positions 1882_1883 of SEQ ID No 2 in the sample; and
c) recording the presence of a AA in the UNC5C gene at position 1882_1883 of SEQ ID No 2 in the sample.

This method is applicable to determine if a human patient is currently suffering from or is likely to develop a solid cancer, such as colorectal cancers, head and neck, thyroid, spinal cord, pancreas, liver, and lung cancer, comprising:
a) analyzing a nucleic acid sample from the human patient;
b) detecting a AA in the UNC5C gene at positions 1882_1883 of SEQ ID No 2 in the sample; and
c) recording the presence of a AA in the UNC5C gene at position 1882_1883 of SEQ ID No 2 in the sample, wherein the presence of a AA at position 1882_1883 of SEQ ID No 2 indicates the human patient is currently suffering from or is likely to develop solid cancer.

The analyzing may comprise:
a) sequencing a region of the nucleic acid sample comprising positions 1882 1883 of SEQ ID No 2; or
b) hybridizing under stringent conditions the nucleic acid sample with at least one probe comprising at least 10 consecutive nucleotides of SEQ ID No 2 comprising position positions 1882_1883 of SEQ ID No 2.

The analysis may also comprise amplifying a region of the nucleic acid sample comprising positions 1882_1883 of SEQ ID No 2 with at least one pair of primers and hybridizing under stringent conditions the nucleic acid sample with at least one probe comprising at least 10 consecutive nucleotides of SEQ ID No 2 comprising positions 1882_1883 of SEQ ID No 2. The at least one probe is labeled with at least one marker and detecting comprises detection of the signal produced by the at least one probe. The hybridizing mau comprise hybridizing with at least two probes one specific said mutations in positions 1882_1883 and the other one being specific to non mutated sequence at positions 1882_1883 (negative control), both probes being labelled with different markers for discrimination. The nucleic acid sample is preferably mRNA but can also be genomic DNA amplified in its cDNA and wherein amplifying comprises amplifying by reverse transcriptase-polymerase chain reaction.

To put it in other words, the invention is directed in vitro method for diagnosis solid cancer or predisposition to solid cancer comprising determining the presence of absence of the 1882_1883GC>AA mutation in the UNC5C gene as depicted as SEQ ID No 3 or polymorph variants thereof, in a sample taken from a patient suffering from cancer, suspected to suffer from cancer, or likely to develop cancer, the method comprising:
a) obtaining a nucleic acid sample from the patient, b) detecting the presence or absence of the the 1882_1883GC>AA mutation in the UNC5C gene in said nucleic acid sample, **characterized in that** the presence of the G1849T variant is an indication of predisposition to solid cancer, especially colorectal cancer when found in any tissue of said patient (germinal mutation) or is an indication of cancer when found in tissue suspected to be tumoral (somatic or germinal mutation).

The nucleic acid sample may be obtained from any cell source or tissue biopsy. These cells may be from any tissue or any corporal fluid. The DNA can be extracted using any known method in the state of the art such as the methods described in Sambrook et al (1989). The RNA can also be isolated, for example from tissues obtained during a biopsy, using standard methods well known to those skilled in the art, such as extraction by guanidiumthiophenate-phenol-chloroform.

The 1882_1883GC>AA mutation in the UNC5C gene can be detected in a RNA or DNA sample, preferably after amplification For example, the isolated RNA can be subjected to reverse transcription followed by amplification, such as a RT-PCR reaction using oligonucleotides specific to the mutated site or which allow amplification of the region containing the mutation, for example exon 11 of the UNC5C gene or sequence SEQ ID No 4. The expression "oligonucleotide" is used here to designate a nucleic acid of at least 10, preferably between 15 and 25 nucleotides, preferably less than 100 nucleotides, and which is able to hybridise to the genomic DNA of UNC5C, to cDNA or to mRNA.

The oligonucleotides of the invention may be labelled using any technique known to those skilled in the art, such as radioactive, fluorescent or enzymatic labellers. A labelled oligonucleotide can be used as a probe to detect the presence or absence of the The 1882_1883GC>AA mutation in the UNC5C gene.

The probes and primers for use in the invention are those which hybridise specifically to the region of the UNC5C gene in the vicinity of the nucleotides at positions 1882 and 1883 (numbering as from the ATG marking the start of transcription).

In the above-explained method, the nucleic acids may be PCR amplified before detection of the allelic-variation. The methods for detecting the allelic variation are described for example in "Molecular Cloning--A Laboratory Manual" Second Edition, Sambrook, Fritsch and Maniatis (Cold Spring Harbor Laboratory, 1989) and Laboratory Protocols for Mutation Detection, Ed. U. Landegren, Oxford University Press, 1996, and PCR 2.sup.nd edition by Newton & Graham, BIOS Scientific Publishers Limited 1997.

In this respect it is possible to combine an amplification step followed by a detection step allowing discrimination between the samples in relation to the presence or absence of said variant. Different techniques adapted for this purpose are described in EP 1 186 672 such as DNA sequencing, sequencing by SSCP, DGGE, TGGE hybridisation, heteroduplex analysis, CMC, enzymatic mismatch cleavage, hybridisation based solid phase hybridisation, DNA chips, Taqman.TM. hybridisation phase solution (U.S. Pat. No. 5,210,015 and U.S. Pat. No. 5,487,972) and the RFLP technique.

Detection can be conducted using different alternative methods: FRET, fluorescence quenching, polarised fluorescence, chemiluminescence, electro-chemiluminescence, radioactivity and colorimetry.

The method of the invention can include or exclude the steps consisting of obtaining the sample and extracting the nucleic acid from said sample.

Therefore, in one particular embodiment, the method of the invention may comprise the amplification step with said primers, followed by an hybridisation step with at least one probe, preferably two probes which hybridise under conditions of high stringency to the sequences corresponding to the region of the described above 1882_1883GC>AA mutation, and detection of the signal produced by the labellers of said probes.

This detection by means of SNPs may be implemented using Taqman.RTM. Technology enabling allelic discrimination. Essentially, this method consists of the recognition, by the fluorescent probes specific to alleles 1882 and 1883 of UNC5C on single strand DNA and comprises a PCR reaction (with a polymerase with 5' exonuclease activity), detection of fluorescence emission specific to the allele of the hybridised SNPs, determination of the genotype by reading end point fluorescence (obtaining an image showing clusters of mutated homozygous, heterozygous and normal DNA).

Is is contemplated that the method further comprises screening or detecting variants in UNC5A (NCBI accession number NM_133369 - SEQ ID No 10) and UNC5B (NCBI accession number NM_170744 - SEQ No 11), such as the UNC5A V5031 variant.

The above method may further comprise detection of alterations in other risk genes such as BRCA1, BRCA2, p53, CDKN2A, MMR, APC, CYLD, PTCH, VHL, WT1, PTENRB, MUTYH, ATM, BLM, FANC, NBS1, RTS, WRN, XP, KIT, MET, PDGFRa and RET.

### 4 - Detection of the Mutated Protein UNC5C A628K

According to another embodiment, the variant can be detected directly within the UNC5C protein.

For this purpose, the invention concerns an ex vivo or in vitro method for detecting the presence or absence of the UNC5C A628K protein variant in a sample from a patient suffering from or likely to develop solid cancer, consisting of detecting the presence or absence of the UNC5C A628K variant, **characterized in that** the presence of said variant is an indication of solid cancer or predisposition to solid cancer, such as colorectal cancer.

Said UNC5C A628K variant can be detected by any suitable method known in the state of the art. More particularly, a sample taken from an individual can be contacted with an antibody specific to the A628K variant of the UNC5C protein, e.g. an antibody which is able to distinguish between the A628K variant and the non-mutated UNC5C protein (and any other protein).

The antibodies of the present invention can be monoclonal or polyclonal antibodies, single chain or double chain, chimeric or humanised antibodies or portions of immunoglobulin molecules containing the portions known in the state of the art to correspond to the antigen binding fragments [human fragment, human F(ab')2 and F(v)]. These antibodies may be immunoconjugated, for example with a toxin or a marker.

The protocols for obtaining polyclonal antibodies are well known to those skilled in the art. Typically, said antibodies can be obtained by administering the UNC5C A628K variant via subcutaneous injection into white New Zealand rabbits previously prepared to obtain a pre-immunity serum. The rabbits are prepared two weeks before the first injection, then periodically stimulated with the same antigen approximately three times every six weeks. A serum sample is then obtained ten days after each injection. The polyclonal antibodies are then purified of the serum by affinity chromatography using the UNC5C A628K protein to capture the antibodies.

Monoclonal antibodies are preferred to polyclonal antibodies on account of their high specificity.

Obtaining said monoclonal antibodies is within the reach of persons skilled in the art bearing in mind that the UNC5C A628K variant has a different 3D structure to the wild-type UNC5C protein. The expression "monoclonal antibody" means an antibody which is able to recognize only an epitope of an antigen.

Monoclonal antibodies can be prepared by immunizing a mammal, e.g. a mouse, rat or other mammals with the purified UNC5C A628K variant. The cells of the immunised mammal producing the antibodies are isolated and fused with the cells of myelomas or hetero-myelomas to produce hybrid cells (hybridomas).

The hybridoma cells producing the monoclonal antibody are used as production source for the antibody. The techniques for generating antibodies which do not involve immunisation are also concerned by the invention. For example "phage display" technology.

The antibodies directed against the UNC5C A628K variant may in some cases show a cross reaction with the wild-type UNC5C protein. If this is the case, a selection of the antibodies specific to the A628K variant is required. In this respect affinity chromatography may be used for example with the wild-type UNC5C protein to capture the antibodies showing a cross reaction with wild-type UNC5C.

Therefore, the invention relates to a monoclonal antibody specifically recognizing the UNC5C A628K variant and to the hybridoma lines producing said antibody.

The invention also concerns an ELISA test using said antibody to detect the presence or absence of the UNC5C A628K variant in a sample.

One alternative to the use of antibodies may for example consist of preparing and identifying haptamers which are classes of molecules enabling specific molecular recognition.

Haptamers are oligonucleotides or oligopeptides which can virtually recognize any class of targeted molecules with high affinity and specificity.

It is within the scope of the invention that the above embodiment be practiced to detect other variants such as P57T, R603C, Q630C A841Y, or A628E.

### 5 - Kits

According to still another feature, the invention relates to kits to determine whether a patient is suffering or likely to suffer from solid cancer, in particular colorectal cancer, as well as head and neck, thyroid, spinal cord, pancreas, liver, or lung cancer, involving the UNC5C A628K variant.

The kit of the invention may contain one or more probes or primers such as defined above for the specific detection of the presence or absence of the 1882_1883GC>AA mutation in the UNC5C gene.

For example, the kit may comprise primers to amplify the UNC5C gene for screening mutations or alterations in the all gene sequence. It may comprise primers to amplify exon 11 of UNC5C or a region at the vicinity of the mutation 1882_1883GC>AA.

It is within the scope of the invention to provide the above kit firther comprising specific probes and/or primers to detect alterations including any alteration in position 628 including A628E or A628K and/or,any alerations resulting in loss of apoptotic function such as UNC5C P57T, R603C, Q630C, or A841Y .

Is is contemplated that the kit further comprise reagents to screen or detect variants in UNC5A (NCBI accession number NM_133369 - SEQ ID No 10) and UNC5B (NCBI accession number NM_170744 - SEQ No 11), such as the UNC5A V5031 variant.

The kit may also contain a heat-resistant polymerase for PCR amplification, one or more solutions for amplification and/or the hybridisation step, and any reagent with which to detect the marker.

The kit may also comprise the UNC5C gene sequence comprising all or parts of SEQ ID No 3 as control and if necessary all or parts of SEQ ID No 10 and 11 (UNC5A and 5B) as control.

According to another embodiment, the kit contains an antibody such as defined above.

The kits of the invention may also contain any reagent adapted for hybridisation or immunological reaction on a solid carrier.

The method and the detection kit are advantageously used for the sub-population of patients that are relatives in kindred with complex familal cancer cases such as in particular colorectal cancer, as well as head and neck, thyroid, spinal cord, pancreas, liver, or lung cancer.

For the remainder of the description and for the examples reference is made to the figures whose keys are described below:

### KEYS TO FIGURES

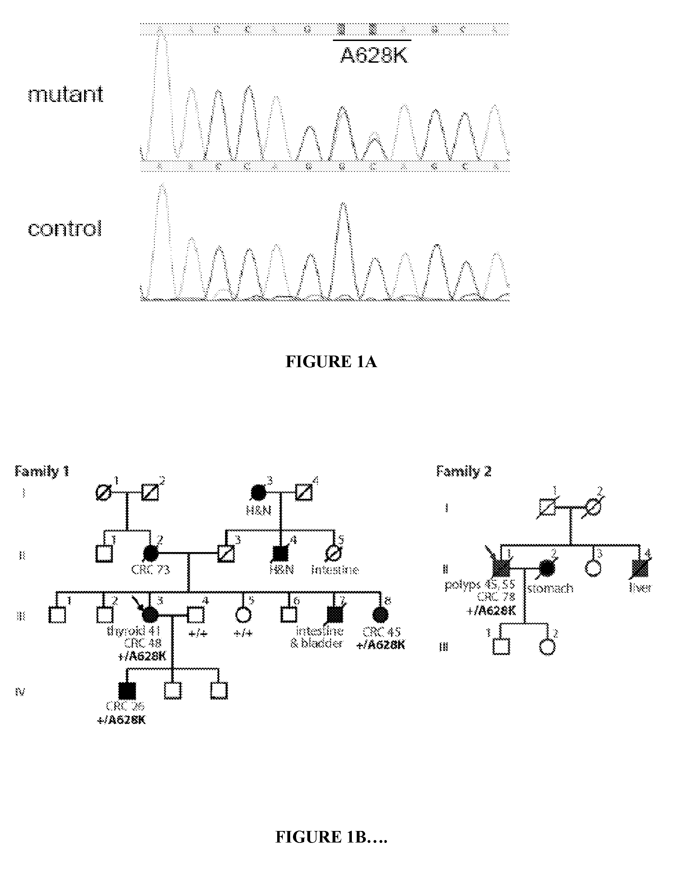

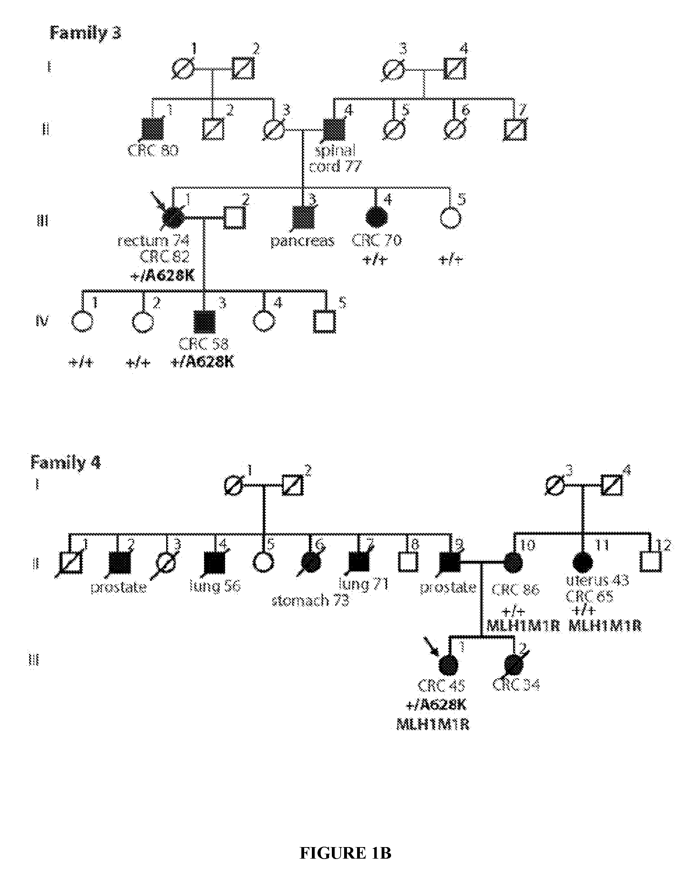

**FIG. 1****: A628K mutation in families with hereditary colorectal cancer** a, Four-color-sequencing chromatograms from automated DNA sequencer showing the germinal missense mutation in the UNC5C gene from a patient (patient III:3 Family 1 in Fig.1b), and a control sequence (Caucasian control population). Coding strand is shown. M: C and A, R: G and A. b, Pedigree of the four families. The probands screened for UNC5C mutations are indicated by arrows. Individuals with cancer are shown as filled circles/squares. Different cancers are indicated beneath the relevant individuals, with age of the diagnosis next to the cancer type. +/+, no sequence variation observed; +/A628K, mutation in UNC5C gene; MLH1M1R, mutation in gene MLH1 (p.Met1Arg), are indicated beneath individuals. Absence of a genotype indicates that DNA could not be obtained from the corresponding individual. We obtained informed consent from all families. H&N (Head&Neck cancer). Roman numerals refer to generations, and individuals within a generation are numbered from left to right, as per convention.
F**IG. 2: Haplotyping of individuals in family 1 and family 3.** a, Map of the UNC5C locus indicating the approximate position of the different SNP markers in this region. Majority are intronic markers. The position of the more telomeric markers is indicated. b, Family pedigrees of individual genotyped in family 1 and 3. Numerals refer to generations and individuals within the generation as in figure 1b. SNP markers name and position towards the A628K mutation in exon 11 are given on the left. Arrows indicate the markers located in exons in UNC5C gene. Alleles are presented beneath each individual and genotypes are depicted. A common disease haplotype (red) segregated with germline transmission of A628K mutation on an interval of 108692 bp. Apart from this common haplotype, the distance on which markers have been analysed is given.

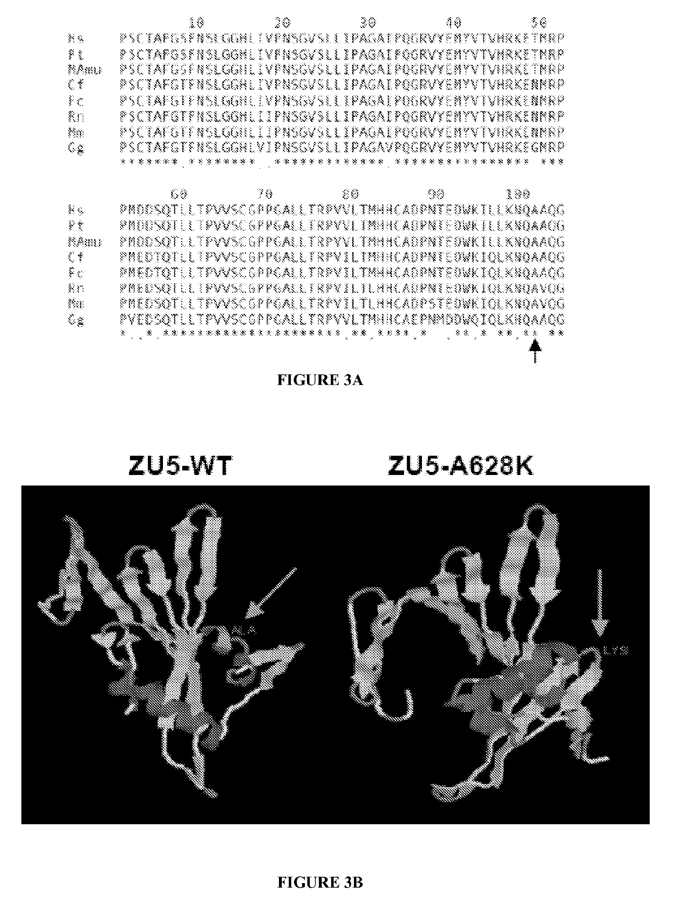

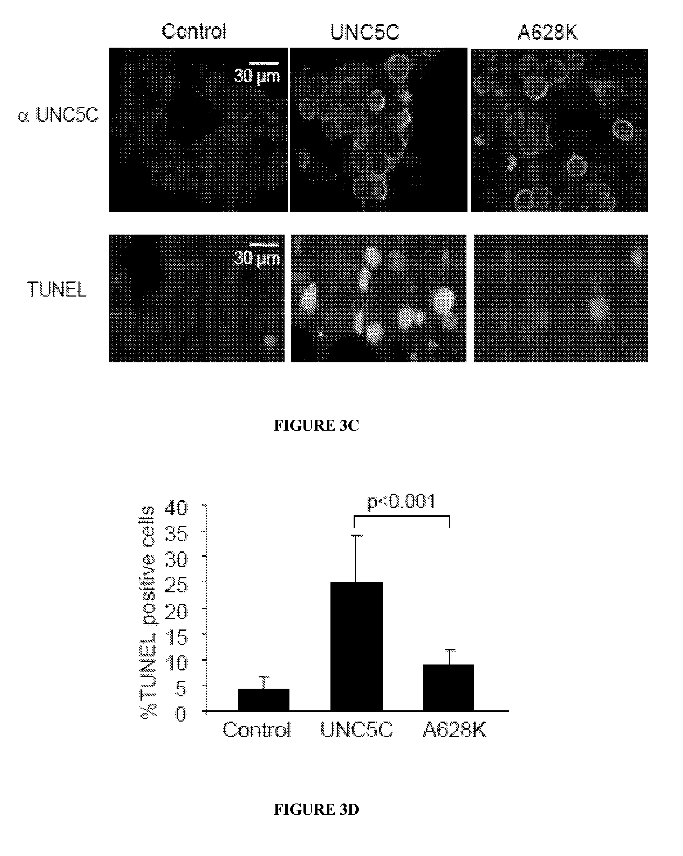

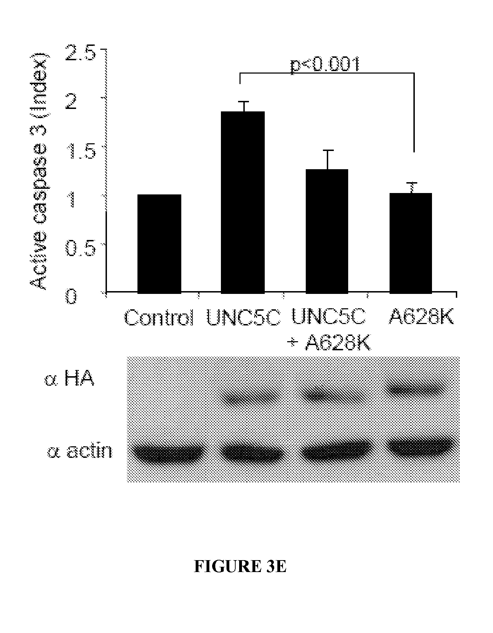

**FIG. 3****: The A628K mutation is a loss-of pro-apoptotic function mutation.** a, Alignment sequence of the UNC5C ZU5 domain among species. Arrow indicates A628. Hs: Homo sapiens, Pt: Pan troglodytes, MAmu: Macaca mulata, Cf: Canis familiaris, Fc: Felix cattus, Rn: Rattus norvegicus, Mm: Mus musculus and Gg: Gallus gallus. b, Putative ab initio 3D structure of the ZU5 domain of human UNC5C using I-sites HMMSTR and Rosetta 18. The alpha-helices are in pink and beta sheets are in yellow. The amino acid 628 is indicated with a green arrow in the wild-type domain (ALA) and in the mutant domain (LYS). c,d,e, Mock vector (control) or constructs containing missense mutation within the ZU5 domain of UNC5C (A628K) or wild-type UNC5C (UNC5C) were transiently transfected separately or in equimolar concentration in HEK293T cells. c, lmmunostaining on HEK293T cells using UNC5C antibody and confocal microscopy. d, Apoptosis quantification by TUNEL staining. Scale bars are indicated. e, Apoptosis was also quantified by measurement of caspase 3 activation. Percentage of cells with active caspase-3 is shown. Below panel: Western blot using anti-HA was performed after transient transfection in HEK293T cells. In d and e. Standard deviations are indicated (resp. n=3 and 7). Statistical analysis, student test, p are indicated.
**FIG. 4****:** Displayed is the mutation 1882_1883 GC>AA in UNC5C human gene sequence - GENEBANK NCBI NM_003728.2 (as numbered from the first ATG)- SEQ NO 2 (or position 2036-2037 of SEQ ID NO 2 from the first nucleotide)

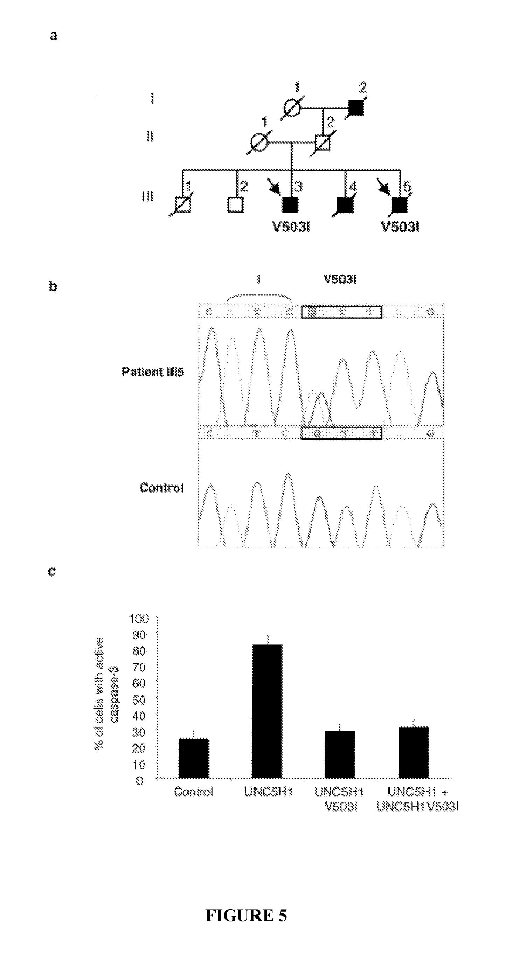

**FIG. 5** : UNC5H1 (UNC5A - GENEBANK NCBI NM_133369) mutation in a family with HNPCC. a, Pedigree of the UNC5H1 mutated family. Roman numerals refer to generations, and individuals within a generation are numbered from left to right, as per convention. Filled and open symbols denote affected and unaffected individuals, respectively; slash line, deceased; arrows indicate that DNA has been analyzed and the genotype for the heterozygous mutation V5031 is shown. Individual 1:2 died from a stomach carcinoma at 45 years old. Individual 11:2 died from an accident at the age of 50 years. Siblings III:3, III:4 and III:5 were found to have colon adenocarcinomas at 70, 54 and 55 years old, respectively. Sibling III.5 died after lung adenocarcinoma at 62 years old. b, Four colour-sequencing chromatograms from automated DNA sequencer (genome express) showing the germinal missense mutation in the UNC5H1 gene from the patient III-5 (R: G and A) and a normal control individual. c, Mock vector (control) or constructs containing missense mutation within the ZU5 domain of UNC5H1 (UNC5H1 V503l) or wild-type UNC5H1 (UNC5H1) were transiently transfected separately or in equimolar concentration in HEK293T cells. Cell death was quantified by measurement of caspase 3 activation. A percentage of cells with active caspase-3 is shown. Standard deviations are indicated (n=5).
FIGURE 6 : TRANSGENIC MOUSE UNC5C A628K

### Example 1 : Identification of the implication of mutations of UNC5C in solid cancers in human

### Methods:

### Individuals and clinical samples.

We obtained genomic DNA from blood from 328 individuals with familial form of colorectal cancers (258 from the Centre Leon Bérard-HEH, France, 60 from the CHU Strasbourg, France and 10 from the previously described CCREF cohort 12), representing unrelated families. Informed consent for cancer genes predisposition research was obtained from all patients. All families present at least two siblings with CRC, except for family 2 for which pedigree informations are lacking. Individuals without familial history were also tested, but no mutation in UNC5C was detected. Controls DNA correspond to 100 individuals of a Portuguese cohort that was a kind gift of Pr. J. Sequeiros (University of Porto, Portugal) and from 1031 individuals of the CCREF cohort 12 for ethnically matched comparison.

### Quantitative RT-PCR:

To assay UNC5H3 expression in human colorectal tumours, total RNA was extracted from biopsies of patients undergoing surgery for colorectal cancer using the Nucleospin RNAII kit (Macherey-Nagel) and 1 µg was reverse-transcribed using the iScript cDNA Synthesis kit (BioRad)(SuperScript II Reverse Transcriptase Rnase H-(Invitrogen)). Real-time quantitative RT-PCR was performed on a LightCycler 2.0 (Roche), using the Light Cycler FastStart DNA Master SYBERGreen I kit (Roche). Reaction conditions for all optimal amplifications, as well as primer selection of UNC5H3, were determined as already described. The ubiquitously expressed RxR , PPIA and GAPDH genes showing the less variability in expression between normal and colorectal tumoral tissues were used as internal controls. Moreover, 8 other ordinarily housekeeping genes were also used to strengthen the results: beta-actin, Phosphoglycerokinase 1(PGK), beta2-microglobulin, Hypoxanthine ribosyltransferase (HPRT), TATA-box-binding protein (TBP), Porphobilinogen deaminase (PBGD), Transferin receptor (TfR), ribosomal protein large P0 (RPLP0) 31.

### UNC5C DNA sequencing and Haplotype analysis.

Genomic DNA (50ng) was amplified by PCR using primers encompassing the exons 9-11 (ZU5 domain) and 15, 16 (Death domain) of UNC5C with HotStarTaq (Qiagen). PCR products were then sequenced by using forward and reverse primers by Genome express (Grenoble) or screened using endo-1 to detect heteroduplex 13. Markers were designed with Hapmap and UCSC databases for haplotype analysis.

### MSI and MMR genes mutation status.

The MSI status was analysed by PCR amplification of microsatellite markers. Extracted DNAs from tumors and matched normal mucosae were PCR-amplified at five microsatellites loci to evaluate the MSI. The markers correspond to the panel recommended by the National Cancer Institute 14. To detect putative mutations in MMR genes, MLH1, MSH2 and MSH6 genes (exons and intron/exon junctions) were sequenced in collaboration with the Molecular Oncology Unit, Centre Léon Berard.

### Cell line, transfection procedure, cell death assays.

Transient transfections of human embryonic kidney cells (HEK293T) were performed using Lipofectamine reagent according to manufacturer's instructions (Invitrogen). For detection of DNA fragmentation, Terminal deoxynucleodityl transferase mediated dUTP-biotin Nick End Labelling (TUNEL) was performed with 300U/mL TUNEL enzyme (300U/mL) and 6 µM biotinylated dUTP (Roche Diagnostics), and detected with avidine coupled Cy3. Caspase-3 activity was measured using ApoAlert CPP32 kit from Clontech (USA) 15 16.

### Plasmid constructs.

pcDNA3-UNC5C-HA containing human UNC5C full-length coding sequences were used as templates for insertion of mutation using the Quick-change strategy. Insertion of the A628K mutation in UNC5C was performed using the following primers: U3A628KFor 5' CAGCTCAAAAACCAGAAAGTGCAGGGACAATGG 3' SEQ ID No 5 and U3A628KRev 5' CCATTGTCCCTGCACTTTCTGGTTTTTGAGCTG 3' SEQ ID No 6

### lmmunohistochemistry and immunoblot.

HEK293T were fixed in 4% paraformaldehyde. Coverslides were then hybridized one hour at room temperature with an antibody against human UNC5C (1/500, R&D System). After rinsing in Phosphate Buffer Saline, slides were incubated with an Alexa-488-Donkey anti-Mouse antibody (1/500, Molecular Probes) and nuclei were stained with Hoescht. Slides were analysed using confocal microscopy (Leica TCS SP2). Immunoblot was performed as described previously using anti-HA antibody 17.

### Results

we analysed 328 blood samples of independent individuals with familial histories of CRCs. Mutation screening was realized by either direct sequencing and/or using a mutation mapping endonuclease-based method for exons 9-11 and 15, 16, coding for the intracellular ZU-5 and Death domains of UNC5C, respectively (two domains that have been shown to be required for the pro-apoptotic activity of UNC5C (8). In different index individuals from 4 different families with different origins in France and Portugal, we identified, at a heterozygous state the same dinucleotide missense mutation, due to a G>A transition followed by a C>A transversion (c.1882_1883GC>AA) in exon 11 that causes the amino acid change p.Ala628Lys (A628K) (**Fig. 1a**). Analysis of the four family histories revealed that hereditary colorectal cancer syndrome has been systematically associated with other phenotypic cancer manifestations not typically related in classical CRC syndromes such as head and neck, thyroid, spinal cord, pancreas, liver, or lung cancer (See **Fig. 1b**).

To further search for co-segregation of the variant with the disease, we obtained DNA samples from other affected or unaffected family members for 3 out of the 4 families and identified the mutation carriers. In each kindred available, we were able to demonstrate association of the A628K mutation with CRCs, while we did not observe the mutant allele in unaffected individuals (**Fig.1b**). All the individuals tested followed association of the mutation with CRC except the individuals III.4 (family 3), 11.10 and II.11 (family 4). The individual III.4 developed a CRC at 70 years old, and this is suggestive for the presence of a phenocopy in the kindred (i.e., the lifetime risk of colorectal cancer in the average-risk person, defined as without personal or family history of colorectal cancer and above the age of 50, is 5%-6% 9). Moreover, the CRCs in individuals 11.10 and II.11 (family 4) are probably related to the detected mutation in the MLH1 DNA mismatch repair (MMR) gene, one of the most frequently mutated gene found in hereditary nonpolyposis colorectal cancer (HNPCC) (10).

In contrast, analysis of 1131 ethnically matched controls (not relative) not affected by any cancer (2262 chromosomes) revealed no example of the mutant allele (data not shown). Because the A628K mutation occurs at a much higher frequency in CRC-prone families (4 mutations in 328 (1.2 %)) compared with control (p=0.002615 by Fisher's exact test), we concluded that this UNC5C variant is associated with a predisposition to CRC cancer, either by itself and/or in combination with other predisposing mutations. Along this line and as mentioned above, in the family 4 the proband (III.1 ), her mother (II.10) and the sister of her mother (II.11) who are CRC-affected, also display a germline mutation in the MLH1 gene. However, this association is definitely not a pre-requisite, as no mutations were detected in the probands of the 3 other families in the most common MMR genes (i.e., MLH1, MSH2 and MSH6). Moreover, the tumour of the individual III.3 in the family 1 (see **Fig. 1b**) is genetically stable (microsatellite stable, MSS) supporting an absence of mutation in MMR genes that would result in a MSI (microsatellite instability) phenotype. However, we do not exclude that mutations in other known or unknown genes predisposing to CRC or to the different types of cancer observed in these families might be associated with the phenotypes presented here.

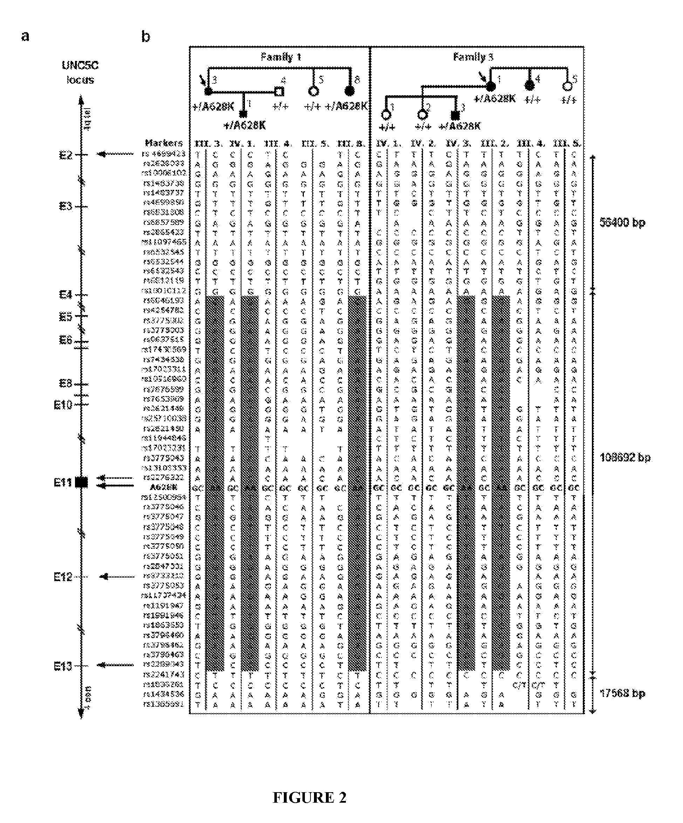

Because these four families with UNC5C A628K were not regionally related, we searched whether this mutation was related to a putative hotspot of mutation or whether this variation was a founder mutation. Haplotype analysis was conducted with 56 polymorphic SNP markers spanning about 183 kbp within the UNC5C locus (**Fig. 2a**). Haplotype profiles of families 1 and 3 are shown in **Fig. 2b** and we identified a shared region encompassing 108692 bp around the A628K mutation suggesting that the mutation probably arose from a common founder. Using the likelihood based method developed by Genin and colleagues 11, the age of the most recent common ancestor of the A628K mutation was estimated to be 13 generations (95% confident interval (CI = 5-33)). Assuming that one generation is 25 years, this corresponds to 450 years (95% confident interval (CI = 125-825)).

Because this common variant appears to be associated with the development of CRCs and is located within the intracellular ZU5 pro-apoptotic domain of the UNC5C receptor, we then analyzed whether this allelic variant was a loss-of-function. Sequence comparisons of UNC5C from different species show that the amino-acid A628 is conserved among species (**Fig. 3a**) and appears as a key amino acid in the structure. Indeed, we predicted that the A628K change prevents the formation of one out of the three alpha-helices implicated in the three-dimensional (3D) structure of the ZU5 domain (**Fig. 3b**). Thus, we assessed whether the A628K mutation in UNC5C somehow modulates its dependence receptor activity -i.e., its ability to trigger apoptosis when expressed in absence of its ligand. HEK293T cells were transiently transfected with constructs encoding wild-type UNC5C, mutant UNC5C A628K, or an equimolar concentration of both plasmids (**Fig. 3cde**). Both wild-type UNC5C and A628K mutant were expressed at the membrane after transfection, as shown by immunohistochemistry (**Fig. 3c**). Cell death was then analysed, either by TUNEL assay (**Fig. 3d**), or by measuring caspase activation (**Fig. 3e**). While wild-type UNC5C receptor is pro-apoptotic in absence of its ligand, the UNC5C A628K mutant is no more effective in triggering apoptosis. Moreover, it is interesting to note that the presence of only half of the total UNC5C protein under the mutated form is sufficient to strongly reduce the ability of UNC5C to induce cell death (**Fig. 3e**). Together with the observation that, in mice, inactivation of UNC5C (rcm) is associated with intestinal tumour progression 4, these in vitro data suggest that patients with one loss-of-function mutation in the ZU5 domain of UNC5C may have an increased likelihood of developing colorectal cancer.

We present here the first genetic evidence that a germline mutation in UNC5C (**Fig. 4**) has a role in CRCs susceptibility. The observation that a loss-pro-apoptotic function UNC5C mutation is associated with the development of cancer in CRC families further strengthens the dependence receptor notion and demonstrates per se that these receptors participate in the control of tumour development. Although almost all the major-gene influences on CRCs were thought to be identified already, the detection of a mutation in the UNC5C gene in 1.2 % of hereditary CRC families calls for a larger screen for CRC-associated mutations in other netrin-1 dependence receptors-induced cell death pathways.

At last, we have shown here that it would be a selective advantage for a tumour cell to lose UNC5H3 in order to survive in a setting of netrin-1 limitation. This selective advantage in the human pathology can be achieved at low frequency by LOH or at a much larger extent by promoter methylation. Moreover, in addition to UNC5C inactivation by loss of expression, UNC5C inactivation by loss-of function might have been selected through mutations that possibly turn down UNC5C pro-apoptotic activity. In any case, the detection of an HNPCC-associated mutation in the UNC5C gene calls for a larger screen for HNPCC-associated mutations in netrin-1 dependence receptors-induced cell death pathways. Along this line, we detected a missense mutation (V5031) in the ZU5 domain of UNC5A in two patients of a family diagnosed with HNPCC without mutations in MMR genes (Fig. 5). Similarly to the mutation found in UNC5C, this mutation abolishes UNC5A pro-apoptotic activity, hence supporting further the implication of netrin-1 dependence receptors in the hereditary nonpolyposis colon cancer syndrome.

### References

1 Aaltonen, L. et al., Explaining the familial colorectal cancer risk associated with mismatch repair (MMR)-deficient and MMR-stable tumors. Clin Cancer Res 13 (1), 356 (2007).
2 Lichtenstein, P. et al., Environmental and heritable factors in the causation of cancer--analyses of cohorts of twins from Sweden, Denmark, and Finland. N Engl J Med 343 (2), 78 (2000).
3 Mazelin, L. et al., Netrin-1 controls colorectal tumorigenesis by regulating apoptosis. Nature 431 (7004), 80 (2004).
4 Bernet, A. et al., Inactivation of the UNC5C Netrin-1 receptor is associated with tumor progression in colorectal malignancies. Gastroenterology 133 (6), 1840 (2007).
5 Shin, S. K. et al., Epigenetic and genetic alterations in Netrin-1 receptors UNC5C and DCC in human colon cancer. Gastroenterology 133 (6), 1849 (2007).
6 Mehlen, P. and Bredesen, D. E., The dependence receptor hypothesis. Apoptosis 9, 37 (2004).
7 Mehlen, P. and Puisieux, A., Metastasis: a question of life or death. Nat Rev Cancer 6 (6), 449 (2006).
8 Williams, M. E., Strickland, P., Watanabe, K., and Hinck, L., UNC5H1 induces apoptosis via its juxtamembrane domain through an interaction with NRAGE. J Biol Chem 278 (19), 17483 (2003); Llambi, F., Causeret, F., Bloch-Gallego, E., and Mehlen, P., Netrin-1 acts as a survival factor via its receptors UNC5H and DCC. Embo J 20 (11), 2715 (2001).
9 Rustgi, A. K., The genetics of hereditary colon cancer. Genes Dev 21 (20), 2525 (2007).
10 Lynch, H. T. and de la Chapelle, A., Hereditary colorectal cancer. N Engl J Med 348 (10), 919 (2003).
11 Genin, E. et al., Estimating the age of rare disease mutations: the example of Triple-A syndrome. J Med Genet 41 (6), 445 (2004).
12 Andrieu, N. et al., Familial relative risk of colorectal cancer: a population-based study. Eur J Cancer 39 (13), 1904 (2003).
13 Triques, K. et al., Mutation detection using ENDO1: application to disease diagnostics in humans and TILLING and Eco-TILLING in plants. BMC Mol Biol 9, 42 (2008).
14 Boland, C. R. et al., A National Cancer Institute Workshop on Microsatellite Instability for cancer detection and familial predisposition: development of international criteria for the determination of microsatellite instability in colorectal cancer. Cancer Res 58 (22), 5248 (1998).
15 Mehlen, P. et al., The DCC gene product induces apoptosis by a mechanism requiring receptor proteolysis. Nature 395 (6704), 801 (1998).
16 Thibert, C. et al., Inhibition of neuroepithelial patched-induced apoptosis by sonic hedgehog. Science 301 (5634), 843 (2003).
17 Llambi, F. et al., The dependence receptor UNC5H2 mediates apoptosis through DAP-kinase. Embo J 24 (6), 1192 (2005).
18 Bystroff, C. and Shao, Y., Fully automated ab initio protein structure prediction using I-SITES, HMMSTR and ROSETTA. Bioinformatics 18 Suppl 1, S54 (2002).

## Claims

1. An in vitro method to determine if a human patient is currently suffering from or is likely to develop a solid cancer, such as colorectal cancers, head and neck, thyroid, spinal cord, pancreas, liver, and lung cancer, comprising:
a) analyzing a nucleic acid sample from the human patient;
b) detecting the presence or absence of variants in the UNC5C gene (human unc-5 homolog C, the wild-type sequence of which being displayed as SEQ ID No 3 or polymorphic variant thereof), wherein said variants results in the loss of the pro-apoptotic function of the UNC5C protein and wherein the presence of said variant in said UNC5C gene indicates that the human patient is currently suffering from or is likely to develop solid cancer.

2. The method according to claim 1, comprising the detection of at least one variant selected from P57T, R603C, Q630C A841Y, A628E and A628K in the UNC5C gene as shown in SEQ ID No 9, more specifically at least one mutation selected from one or more mutations selected from 169 C>A , 1807 C>T, 1888 C>G and c.1882_1883GC>AA as shown in SEQ ID No 12.

3. An in vitro method to determine the presence or absence of the 1882_1883 GC>AA UNC5C mutation in the UNC5C gene (human unc-5 homolog C, the wild-type sequence of which being displayed as SEQ ID No 3 or polymorphic variant thereof), which mutation results in the UNC5C A628K variant protein of SEQ ID No 1, in a human patient comprising:
a) analyzing a nucleic acid sample from the human patient;
b) detecting a AA in the UNC5C gene at positions 1882_1883 of SEQ ID No 2 in the sample; and
c) recording the presence of a AA in the UNC5C gene at position 1882_1883 of SEQ ID No 2 in the sample.

4. The in vitro method according to claim 1 or 2 to determine if a human patient is currently suffering from or is likely to develop a solid cancer, such as colorectal cancers, head and neck, thyroid, spinal cord, pancreas, liver, and lung cancer, comprising:
a) analyzing a nucleic acid sample from the human patient;
b) detecting a AA in the UNC5C gene at positions 1882_1883 of SEQ ID No 2 in the sample; and
c) recording the presence of a AA in the UNC5C gene at position 1882_1883 of SEQ ID No 2 in the sample, wherein the presence of a AA at position 1882_1883 of SEQ ID No 2 indicates that the human patient is currently suffering from or is likely to develop solid cancer.

5. The method of claim 3 or 4, wherein the analyzing comprises:
a) sequencing a region of the nucleic acid sample comprising positions 1882_1883 of SEQ ID No 2; or
b) hybridizing under stringent conditions the nucleic acid sample with at least one probe comprising at least 10 consecutive nucleotides of SEQ ID No 2 comprising position positions 1882_1883 of SEQ ID No 2.

6. The method of claim 5, wherein analyzing comprises amplifying a region of the nucleic acid sample comprising position positions 1882_1883 of SEQ ID No 2 with at least one pair of primers and hybridizing under stringent conditions the nucleic acid sample with at least one probe comprising at least 10 consecutive nucleotides of SEQ ID No 2 comprising positions 1882_1883 of SEQ ID No 2.

7. The method of claim 5 or 6, wherein the at least one probe is labeled with at least one marker and detecting comprises detection of the signal produced by the at least one probe.

8. The method of claim 6 or 7, comprising hybridizing with at least two probes.

9. The method of claim 6, wherein the nucleic acid sample is mRNA and wherein amplifying comprises amplifying by reverse transcriptase-polymerase chain reaction.

10. The method according to claims 3 further comprising detecting the presence or absence of at least one variants resulting in the loss of pre-apoptotic function of UNC5C, especially at least one of the following variants : P57T, R603C, Q630C A841Y and A628E alterations in the UNC5C gene as shown in SEQ ID No 9.

11. The method according to one of claims 1 to 10 further comprising detecting the presence or absence of variants altering the function of the UNC5A and UNC5B gene product, especially the V5031 UNC5A variant.

12. A method for screening alterations in the sequence of the UNC5C gene as shown in SEQ ID No 3 or polymorphic variant thereof in a tissue sample of a subject comprising sequence the UNC5C gene of said subject and comparing to the nucleotide sequence of wild type UNC5C gene set forth in SEQ ID No 3 or a wild-tyle allelic variant thereof, said alteration indicating a predisposition to solid cancer.

13. A method according to claim 12 wherein it comprises detecting deletions, insertion or point mutations, especially mutations resulting in frameshift or resulting in the loss of expression or activity of the UNC5C gene product.

14. A primer or probe comprising at least 10 consecutive nucleotides of a region at the vicinity or encompassing the 1882_1883GC>AA mutation as shown in SEQ ID No 2.

15. A primer or probe comprising at least 10 consecutive nucleotides of SEQ ID No 4, such as SEQ ID No 7 and 8.

16. An isolated protein UNC5C (unc-5 homolog C), in particular the Homo sapiens UNC5C (NCBI, accession number NM_003728.2 GI:16933524) comprising the A628K mutation as shown in SEQ ID No 1, or polymorphic variant thereof, called UNC5C A628K variant, or comprising one mutation as shown in SEQ ID No 9.

17. An isolated nucleotide sequence encoding SEQ ID No 1, preferably of SEQ ID No 2, (sequence of the human gene with the AAA codon instead of GCA in codon 628 (a G>A transition followed by a C>A transversion (c.1882_1883GC>AA) in exon 11 that causes the amino acid change p.Ala628Lys (A628K) ; positions 1882 and 1883 are calculated from the ATG marking the start of translation, or encoding a variant of UNC5C bearing one mutation as shown in SEQ ID No 9.

18. A cloning and/or expression vector comprising the sequence defined in claim 17.

19. A non-human transgenic animal expressing a recombinant UNC5C A628K, P57T, R603C, Q630C, A841Y or A628E variant protein.

20. A monoclonal antibody directed against the UNC5C variant protein as defined in claim 19.

21. A kit for screening or diagnosis comprising one or more probes or primers such as probes or primers defined in claim 14 or 15 for the specific detection of the presence or absence of the 1882_1883GC>AA mutation in the UNC5C gene.

22. A kit for screening or diagnosis comprising one or more probes or primers for specific detection of at least one of UNC5C A628K, P57T, R603C, Q630C, A841 Y or A628E variant proteins.
